# EUROPEAN PATENT APPLICATION

(11) **EP 4 310 067 A1**
(43) Date of publication of application: **24.01.2024**
(21) Application number: 22771541.4
(22) Date of filing: 18.03.2022
(51) Int. Cl.: C07B 49/00, C07C 29/40, C07C 33/20, C07C 33/24, C07C 33/34, C07C 33/50, C07C 45/00, C07C 45/61, C07F 3/02, C07C 49/11

(54) **METHOD FOR PRODUCING ORGANIC METAL NUCLEOPHILIC AGENT AND REACTION METHOD USING ORGANIC METAL NUCLEOPHILIC AGENT**

(30) Priority: 19.03.2021 JP 2021046299
(71) Applicant: National University Corporation Hokkaido University, Sapporo-shi, Hokkaido 060-0808 (JP)
(72) Inventor: ITO, Hajime, Sapporo-shi, Hokkaido 060-0808 (JP); KUBOTA, Koji, Sapporo-shi, Hokkaido 060-0808 (JP)
(74) Representative: Grünecker Patent- und Rechtsanwälte PartG mbB
(86) International application number: PCT/JP2022/012524
(87) International publication number: WO 2022/196796

(57) **Abstract**

A first object is to provide a method for producing an organometallic nucleophile, which uses a reduced amount of organic solvent to reduce environmental load and which does not require the adjustment of an atmosphere or water (moisture) and thus is a simplified means without the use of a large-scale apparatus. A second object is to provide a reaction method by which reactions between an organometallic nucleophile and various organic electrophiles can be performed by an efficient and simplified means such as a one-pot system and which is advantageous in terms of cost. A third object is to provide a simplified method for producing an organometallic nucleophile having higher reactivity. As a solution, a method for producing an organometallic nucleophile, including reacting an organohalide and a metal or metal compound with each other by a mechanochemical process in the presence of an ether compound in an amount of 0.5 to 10.0 equivalents relative to 1 equivalent of the organohalide, is provided.

## Description

### Technical Field

The present invention relates to a novel method for producing an organometallic nucleophile, the method utilizing a mechanochemical process. The invention also relates to a reaction method using an organometallic nucleophile, the method utilizing a mechanochemical process.

### Background Art

As the organometallic nucleophiles, for example, Grignard reagents (RMgX) are widely known. Grignard reagents have been one of the most-used carbon nucleophiles in modern organic synthesis since their discovery by Victor Grignard in 1900. In particular, the reaction between a Grignard reagent and a carbonyl compound is a reaction that can be said to be a pioneering carbon-carbon bond forming reaction and is very important in synthesizing alcohols. However, existing methods for producing organometallic nucleophiles such as Grignard reagents and reaction methods using the organometallic nucleophiles have practical problems because they must be performed under strict water-free conditions in inert gases such as nitrogen. Furthermore, these methods are required to use large amounts of organic solvents and have room for improvement from the viewpoint of environmental load reduction.

Organic synthesis reaction methods in which reaction materials are brought into direct contact with each other and no organic solvents are used are environmentally friendly and academically and industrially interesting. As such an organic synthesis reaction method, a mechanochemical process has attracted attention. The mechanochemical process is a method in which solid raw materials are activated to undergo reaction by applying mechanical energy to the solid raw materials by means of, for example, grinding, shearing, impact, or compression.

PTL 1 discloses a method for producing a polymer compound having a repeating unit by performing a reaction between functional groups by a mechanochemical effect.

PTL 2 discloses a method for debrominating an organic bromine compound by mechanochemically processing the organic bromine compound in the presence of an alkali metal hydroxide.

NPL 1 describes that dechlorination of a mixture of an aryl chloride, magnesium, and n-butylamine by a mechanochemical process using a ball mill involves the formation of an intermediary Grignard reagent.

NPL 2 describes that a halogenated naphthalene and magnesium are reacted with each other under solvent-free conditions by a mechanochemical process using a ball mill in a glove box to obtain a Grignard reagent. However, it is described that when this Grignard reagent and a ketone were reacted with each other by a mechanochemical process, an unexpected reaction occurred and only a reaction mixture containing a by-product was obtained.

NPL 3 describes that fluoronaphthalene, magnesium, and iron chloride are reacted with each other under solvent-free conditions by a mechanochemical process using a ball mill in a glove box. However, the yield of binaphthalene, which is a coupling reaction product, is as low as on the order of 20%.

### Citation List

### Patent Literature

PTL 1: Japanese Patent No. 6733985
PTL 2: Japanese Unexamined Patent Application Publication No. 2002-30003

### Non Patent Literature

NPL 1: Fresenius Encironmental Bulletin, Vol. 20, No. 10a, P. 2794-2805 (2011)
NPL 2: Australian Journal of Chemistry, Vol. 54, P. 423-425
NPL 3: Molecules 2020, 25, 570

### Summary of Invention

### Technical Problem

In the fields of pharmaceuticals, organic materials, foods, polymers, etc., organometallic nucleophiles such as Grignard reagents are extremely useful for producing various compounds and intermediates and are widely used.

However, in producing an organometallic nucleophile, it is necessary to create an inert atmosphere using a noble gas or the like in a large-scale hermetically sealed apparatus or to use a large amount of dry organic solvent (anhydrous organic solvent) in order to reduce the influence of oxygen and water (moisture). In addition, it is also necessary to consider, for example, a means for activating a reaction reagent by, for example, removing an oxide film on a metal reagent surface. Furthermore, in handling or storing an organometallic nucleophile, it is necessary to consider, for example, the Schlenk equilibrium. Thus, improvements in environmental load, cost of reaction equipment, etc. have been desired.

In addition, Grignard reagents, while being organometallic nucleophiles with high reactivity, sometimes show low reaction yields depending on the substrate.

A first object of the present invention is to provide a method for producing an organometallic nucleophile, which uses a reduced amount of organic solvent to reduce environmental load and which does not require the adjustment of an atmosphere or water (moisture) and thus is a simplified means without the use of a large-scale apparatus.

A second object of the present invention is to provide a reaction method by which reactions between an organometallic nucleophile and various organic electrophiles can be performed by an efficient and simplified means such as a one-pot system and which is advantageous in terms of cost.

A third object of the present invention is to provide a simplified method for producing an organometallic nucleophile having higher reactivity.

### Solution to Problem

As a result of intensive studies to achieve the above objects, the present inventors have focused on the fact that an organic synthesis reaction by a mechanochemical process using a ball mill or the like can be simply performed without using an organic solvent.

As a result of the studies, the present inventors have found that (a) when a reaction between an organohalide and a metal or metal compound is performed in a ball mill, a target organic nucleophile (organomagnesium nucleophile/Grignard reagent, etc.) can be efficiently produced in air under solvent-free conditions by adding a very small amount of ether compound to the reaction system, (b) the organic nucleophile thus prepared reacts with various electrophiles such as aldehydes and ketones on a one-pot system to provide corresponding products, and (c) the organic nucleophile thus prepared can be applied to various reactions such as a coupling reaction using a Ni catalyst, thereby completing the present invention.

Thus, the present invention provides a method for producing an organometallic nucleophile, a method for producing an alcohol, an addition reaction method, and a coupling method described below.

### [Item 1]

A method for producing an organometallic nucleophile, including reacting an organohalide and a metal or metal compound with each other by a mechanochemical process in the presence of an ether compound in an amount of 0.5 to 10.0 equivalents relative to 1 equivalent of the organohalide.

### [Item 2]

A method for producing an alcohol, including reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding an organic carbonyl compound and performing a reaction by the mechanochemical process.

### [Item 3]

A method for producing an alcohol, including reacting an organohalide, a metal or metal compound, and an organic carbonyl compound with each other by a mechanochemical process in the presence of an ether compound in an amount of 0.5 to 10.0 equivalents relative to 1 equivalent of the organohalide.

### [Item 4]

An addition reaction method including reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding an electrophile and performing a reaction by the mechanochemical process.

### [Item 5]

An addition reaction method including reacting an organohalide, a metal or metal compound, and an electrophile with each other by a mechanochemical process in the presence of an ether compound in an amount of 0.5 to 10.0 equivalents relative to 1 equivalent of the organohalide.

### [Item 6]

A coupling reaction method including reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding a nickel catalyst and a sulfonate ester compound and performing a reaction by the mechanochemical process.

### [Item 7]

A coupling reaction method including reacting an organohalide, a metal or metal compound, and a sulfonate ester compound with each other by a mechanochemical process in the presence of a nickel catalyst and an ether compound in an amount of 0.5 to 10.0 equivalents relative to 1 equivalent of the organohalide.

### [Item 8]

An addition reaction method including reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding a conjugated enone compound and performing a reaction by the mechanochemical process.

### [Item 9]

An addition reaction method including reacting an organohalide, a metal or metal compound, and a conjugated enone compound with each other by a mechanochemical process in the presence of an ether compound in an amount of 0.5 to 10.0 equivalents relative to 1 equivalent of the organohalide.

### [Item 10]

A composition obtained by the method according to Item 1, containing a halogenated organometallic compound and an ether compound, in which a content of the ether compound is 0.5 to 10 equivalents relative to 1 equivalent of the halogenated organometallic compound.

### [Item 11]

A halogenated organometallic compound-ether compound complex obtained by the method according to Item 1, including at least a halogenated organometallic compound and an ether compound, in which the complex is represented by formula (A):

[R¹¹-(MX)ₚ]_{q}-rE (A)

(where R¹¹ is a p-valent organic group derived from the halogenated organometallic compound, M is a metal derived from the halogenated organometallic compound, X is a halogen, and E is the ether compound; a plurality of R¹¹'s, M's, X's, or E's, if present, may be the same or different; and p is an integer of 1 or greater, q is a number of 2 or greater, and r is a number greater than 0).

### [Item 12]

A halogenated organometallic compound-ether compound complex including at least a halogenated organometallic compound and an ether compound, in which the complex is represented by formula (A):

[R¹¹-(MX)ₚ]_{q}-rE (A)

(where R¹¹ is a p-valent organic group derived from the halogenated organometallic compound, M is a metal derived from the halogenated organometallic compound, X is a halogen, and E is the ether compound; a plurality of R¹¹'s, M's, X's, or E's, if present, may be the same or different; and p is an integer of 1 or greater, q is a number of 2 or greater, and r is a number greater than 0).

### [Item 13]

An organometallic nucleophile obtained by the method according to Item 1 using an organohalide having a solubility at 20°C in an ether compound of less than 1.0 mol/L.

### Advantageous Effects of Invention

According to the present invention, a method for producing an organometallic nucleophile, which uses a reduced amount of organic solvent to reduce environmental load and which does not require the adjustment of an atmosphere or water (moisture) and thus is a simplified means without the use of a large-scale apparatus, is provided.

According to the present invention, a reaction method by which reactions between an organometallic nucleophile and various organic electrophiles can be performed by an efficient and simplified means such as a one-pot system and which is advantageous in terms of cost is provided.

According to the present invention, a simplified method for producing an organometallic nucleophile having higher reactivity is provided.

### Brief Description of Drawings

[Fig. 1] Fig. 1 shows Mg-K NEXAFS spectra of PhMgBr prepared mechanochemically in Example 116 and PhMgBr prepared in solution.
[Fig. 2] Fig. 2 shows C-K NEXAFS spectra of PhMgBr prepared mechanochemically in Example 116 and PhMgBr prepared in solution.
[Fig. 3] Fig. 3 shows examples of structures of (PhMgBr)m-nTHF complexes prepared mechanochemically in Example 116.

### Description of Embodiments

Embodiment 1 of the present invention is a method for producing an organometallic nucleophile, including reacting an organohalide and a metal or metal compound with each other by a mechanochemical process in the presence of an ether compound in an amount of 0.5 to 10.0 equivalents relative to 1 equivalent of the organohalide.

Embodiment 2 of the present invention is a method for producing an alcohol, including reacting an organohalide and a metal or metal compound with each other by a mechanochemical process in the presence of an ether compound in an amount of 0.5 to 10.0 equivalents relative to 1 equivalent of the organohalide, and further adding an organic carbonyl compound and performing a reaction by the mechanochemical process.

Embodiment 3 of the present invention is a method for producing an alcohol, including reacting an organohalide, a metal or metal compound, and an organic carbonyl compound with each other by a mechanochemical process in the presence of an ether compound in an amount of 0.5 to 10.0 equivalents relative to 1 equivalent of the organohalide.

Embodiment 4 of the present invention is an addition reaction method including reacting reaction components including an organohalide, a metal or metal compound, an electrophile, and an ether compound in an amount of 0.5 to 10.0 equivalents relative to 1 equivalent of the organohalide by a mechanochemical process.

In Embodiment 4, Embodiment 4-1 of the present invention is an addition reaction method including reacting an organohalide and a metal or metal compound with each other by a mechanochemical process in the presence of an ether compound in an amount of 0.5 to 10.0 equivalents relative to 1 equivalent of the organohalide, and further adding an electrophile and performing a reaction by the mechanochemical process.

In Embodiment 4, Embodiment 4-2 of the present invention is an addition reaction method including reacting an organohalide, a metal or metal compound, and an electrophile with each other by a mechanochemical process in the presence of an ether compound in an amount of 0.5 to 10.0 equivalents relative to 1 equivalent of the organohalide.

Embodiment 5 of the present invention is a coupling reaction method including reacting reaction components including an organohalide, a metal or metal compound, a sulfonate ester compound, an ether compound in an amount of 0.5 to 10.0 equivalents relative to 1 equivalent of the organohalide, and a nickel catalyst by a mechanochemical process.

In Embodiment 5, Embodiment 5-1 of the present invention is a coupling reaction method including reacting an organohalide and a metal or metal compound with each other by a mechanochemical process in the presence of an ether compound in an amount of 0.5 to 10.0 equivalents relative to 1 equivalent of the organohalide, and further adding a nickel catalyst and a sulfonate ester compound and performing a reaction by the mechanochemical process.

In Embodiment 5, Embodiment 5-2 of the present invention is a coupling reaction method including reacting an organohalide, a metal or metal compound, and a sulfonate ester compound with each other by a mechanochemical process in the presence of a nickel catalyst and an ether compound in an amount of 0.5 to 10.0 equivalents relative to 1 equivalent of the organohalide.

Embodiment 6 of the present invention is an addition reaction method including reacting reaction components including an organohalide, a metal or metal compound, a conjugated enone compound, and an ether compound in an amount of 0.5 to 10.0 equivalents relative to 1 equivalent of the organohalide by a mechanochemical process.

In Embodiment 6, Embodiment 6-1 of the present invention is an addition reaction method including reacting an organohalide and a metal or metal compound with each other by a mechanochemical process in the presence of an ether compound in an amount of 0.5 to 10.0 equivalents relative to 1 equivalent of the organohalide, and further adding a conjugated enone compound and performing a reaction by the mechanochemical process.

In Embodiment 6, Embodiment 6-2 of the present invention is an addition reaction method including reacting an organohalide, a metal or metal compound, and a conjugated enone compound with each other by a mechanochemical process in the presence of an ether compound in an amount of 0.5 to 10.0 equivalents relative to 1 equivalent of the organohalide.

Embodiment 7 of the present invention is a composition obtained by the method including reacting an organohalide and a metal or metal compound with each other by a mechanochemical process in the presence of an ether compound in an amount of 0.5 to 10.0 equivalents relative to 1 equivalent of the organohalide. The composition contains a halogenated organometallic compound and an ether compound, and a content of the ether compound is 0.5 to 10 equivalents relative to 1 equivalent of the halogenated organometallic compound.

Embodiment 8 of the present invention is a halogenated organometallic compound-ether compound complex including at least a halogenated organometallic compound and an ether compound. The complex is represented by formula (A):

[R¹¹-(MX)ₚ]_{q}-rE (A)

(where R¹¹ is a p-valent organic group derived from the halogenated organometallic compound, M is a metal derived from the halogenated organometallic compound, X is a halogen, and E is the ether compound; a plurality of R¹¹'s, M's, X's, or E's, if present, may be the same or different; and p is an integer of 1 or greater, q is a number of 2 or greater, and r is a number greater than 0).

Embodiment 9 of the present invention is an organometallic nucleophile obtained by reacting an organohalide having a solubility at 20°C in an ether compound of less than 1.0 mol/L and a metal or metal compound with each other by a mechanochemical process in the presence of an ether compound in an amount of 0.5 to 10.0 equivalents relative to 1 equivalent of the organohalide.

Hereinafter, Embodiments of the present invention will be described in detail.

### [Embodiment 1]

Embodiment 1 of the present invention is a method for producing an organometallic nucleophile, including reacting an organohalide and a metal or metal compound with each other by a mechanochemical process in the presence of an ether compound in an amount of 0.5 to 10.0 equivalents relative to 1 equivalent of the organohalide.

### <Organohalide>

The organohalide used in the method for producing an organometallic nucleophile according to the present invention is a compound (I) represented by formula (I) below.

A¹-Xₘ (I)

(In the formula,
A¹ represents an optionally substituted m-valent aromatic hydrocarbon group, an optionally substituted m-valent aromatic heterocyclic group, an optionally substituted m-valent aliphatic hydrocarbon group, or an optionally substituted m-valent unsaturated aliphatic hydrocarbon group.

Each occurrence of X represents F (fluorine), Cl (chlorine), Br (bromine), or I (iodine).

m is the number of X and represents an integer of 1 or greater.)

A single compound (I) can be used alone, or two or more compounds (I) can be used in combination.

As the compound (I), a commercially available product can be used directly or after purification.

### (A¹ group in formula (I))

### {Optionally substituted m-valent aromatic hydrocarbon group}

The number of carbon atoms in the optionally substituted m-valent aromatic hydrocarbon group represented by A¹ is not particularly limited, and is, for example, 6 to 60, preferably 6 to 40, more preferably 6 to 30.

In the m-valent aromatic hydrocarbon group, m is an integer of 1 or greater, for example, 1 to 10, preferably 1 to 6, more preferably 1 to 4.

For the optionally substituted m-valent aromatic hydrocarbon group represented by A¹, examples of monovalent aromatic hydrocarbon groups where m = 1 include a phenyl group, a naphthyl group, an anthracenyl (or anthracene) group, a phenanthrenyl (or phenanthrene) group, a biphenyl group, a terphenyl group, a pyrenyl (or pyrene) group, a perylenyl (or perylene) group, a triphenylenyl (or triphenylene) group, and a fluorenyl group.

For the optionally substituted m-valent aromatic hydrocarbon group represented by A¹, examples of m-valent aromatic hydrocarbon groups where m is an integer of 2 or greater include groups obtained by removing (m - 1) hydrogen atoms from aromatic rings in the above monovalent aromatic hydrocarbon groups.

### {Optionally substituted m-valent aromatic heterocyclic group}

The number of carbon atoms in the optionally substituted m-valent aromatic heterocyclic group represented by A¹ is not particularly limited, and is, for example, 4 to 60, preferably 4 to 40, more preferably 4 to 30.

In the m-valent aromatic heterocyclic group represented by A¹, m is an integer of 1 or greater, for example, 1 to 10, preferably 1 to 6, more preferably 1 to 4.

For the optionally substituted m-valent aromatic heterocyclic group represented by A¹, examples of monovalent aromatic heterocyclic groups where m = 1 include sulfur-containing heteroaryl groups such as a thiophenyl (thiophene or thienyl) group, a thienylenyl (or thiophenediyl) group, a benzothienyl (benzothiophene) group, a dibenzothienyl (dibenzothiophene) group, a phenyldibenzothienylenyl group, and a dibenzothienylenylphenyl group; oxygen-containing heteroaryl groups such as a furanyl (or furan) group, a benzofuranyl (benzofuran) group, a dibenzofuranyl (dibenzofuran) group, a phenyldibenzofuranyl group, and a dibenzofuranylphenyl group; nitrogen-containing heteroaryl groups such as a pyridyl (or pyridine) group, a pyridylenyl (or pyridinediyl) group, a pyrimidinyl (or pyrimidine) group, a pyrazyl (or pyrazine) group, a quinolyl (or quinoline) group, an isoquinolyl (or isoquinoline) group, a carbazolyl (or carbazole) group, a 9-phenylcarbazolyl group, an acridinyl (or acridine) group, a quinazolyl (or quinazoline) group, a quinoxalyl (or quinoxaline) group, a 1,6-naphthyridinyl group, a 1,8-naphthyridinyl group, and a porphyrin group (or a porphyrin ring); and heteroaryl groups containing two or more heteroatoms (e.g., nitrogen and sulfur), such as a benzothiazolyl (or benzothiazole) group and a benzothiadiazole group. Further examples thereof include a pyrrole group, a silole group, a borole group, a phosphole group, a selenophene group, a germole group, an indole group, an indene group, a benzosilole group, a benzoborole group, a benzophosphole group, a benzoselenophene group, a benzogermole group, a dibenzosilole group, a dibenzoborole group, a dibenzophosphole group, a dibenzoselenophene group, a dibenzogermole group, a dibenzothiophene 5-oxide group, a 9H-fluoren-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluoren-9-one group, an azadibenzothiophene 5,5-dioxide group, a pyridazine group, a triazine group, a phenanthroline group, a pyrazole group, an imidazole group, a triazole group, an oxazole group, an isoxazole group, a thiazole group, an isothiazole group, an oxadiazole group, a thiadiazole group, a benzopyrazol group, a benzoimidazole group, a benzoxazole group, a benzooxadiazole group, a 5,6,7,8-tetrahydroisoquinoline group, and a 5,6,7,8-tetrahydroquinoline group.

For the optionally substituted m-valent aromatic heterocyclic group represented by A¹, examples of m-valent aromatic heterocyclic groups where m is an integer of 2 or greater include groups obtained by removing (m - 1) hydrogen atoms from aromatic rings in the above monovalent aromatic heterocyclic groups. Another example is a benzo[1,2-c:4,5-c']bis[1,2,5]thiadiazole skeleton (benzobisthiadiazole group) .

### {Optionally substituted m-valent aliphatic hydrocarbon group}

The number of carbon atoms in the optionally substituted m-valent aliphatic hydrocarbon group represented by A¹ is not particularly limited, and is, for example, 1 to 60, preferably 1 to 40, more preferably 1 to 30.

In the m-valent aliphatic hydrocarbon group represented by A¹, m is an integer of 1 or greater, for example, 1 to 10, preferably 1 to 6, more preferably 1 to 4.

For the optionally substituted m-valent aliphatic hydrocarbon group represented by A¹, examples of monovalent aliphatic hydrocarbon groups where m = 1 include saturated aliphatic hydrocarbon groups such as alkyl groups and cycloolefin groups.

For the optionally substituted m-valent aliphatic hydrocarbon group represented by A¹, examples of m-valent aliphatic hydrocarbon groups where m is an integer of 2 or greater include groups obtained by removing (m - 1) hydrogen atoms from the above monovalent aliphatic hydrocarbon groups.

The number of carbon atoms in the optionally substituted m-valent unsaturated aliphatic hydrocarbon group represented by A¹ is not particularly limited, and is, for example, 2 to 60, preferably 3 to 40, more preferably 5 to 30.

In the m-valent unsaturated aliphatic hydrocarbon group represented by A¹, m is an integer of 1 or greater, for example, 1 to 10, preferably 1 to 6, more preferably 1 to 4.

For the optionally substituted m-valent unsaturated aliphatic hydrocarbon group represented by A¹, examples of monovalent aromatic hydrocarbon groups where m = 1 include alkenyl groups and alkynyl groups.

For the optionally substituted m-valent unsaturated aliphatic hydrocarbon group represented by A¹, examples of m-valent unsaturated aliphatic hydrocarbon groups where m is an integer of 2 or greater include groups obtained by removing (m - 1) hydrogen atoms from the above monovalent unsaturated aliphatic hydrocarbon groups.

### (Substituent in A¹)

The optional substituent in the optionally substituted m-valent aromatic hydrocarbon group, the optionally substituted m-valent aromatic heterocyclic group, the optionally substituted m-valent aliphatic hydrocarbon group, or the optionally substituted m-valent unsaturated aliphatic hydrocarbon group represented by A¹ is not particularly limited as long as the intended reaction of the present invention can be performed.

Examples of the substituent include one or more selected from the group consisting of alkyl groups having 1 to 24, preferably 1 to 18, more preferably 1 to 12, still more preferably 1 to 8 carbon atoms (e.g., a methyl group, an ethyl group, a n-propyl group, an isopropyl group, a n-butyl group, an isobutyl group, a tert-butyl group, a pentyl group, a hexyl group, and an octyl group); alkoxy groups having 1 to 24, preferably 1 to 18, more preferably 1 to 12, still more preferably 1 to 8 carbon atoms (e.g., a methoxy group, an ethoxy group, a n-propoxy group, an isopropoxy group, a n-butoxy group, an isobutoxy group, a tert-butoxy group, a pentyloxy group, a hexyloxy group, and an octyloxy group); cycloalkyl groups having 3 to 24, preferably 3 to 18, more preferably 3 to 12, still more preferably 3 to 8 carbon atoms (e.g., a cyclopropyloxy group, a cyclobutyloxy group, a cyclopentyloxy group, and a cyclohexyloxy group); alkenyl groups having 1 to 24, preferably 1 to 18, more preferably 1 to 12, still more preferably 1 to 8 carbon atoms (e.g., an ethenyl group, a propenyl group, a butenyl group, a pentenyl group, a hexenyl group, and an octenyl group); alkynyl groups having 1 to 24, preferably 1 to 18, more preferably 1 to 12, still more preferably 1 to 8 carbon atoms (e.g., an ethynyl group, a propynyl group, a butynyl group, a pentynyl group, a hexynyl group, and an octynyl group); aryl groups having 5 to 24, preferably 5 to 18, more preferably 5 to 12, still more preferably 5 to 8 carbon atoms (e.g., a phenyl group, a naphthyl group, and a biphenyl group); arylalkyl groups having 7 to 24, preferably 7 to 19, more preferably 7 to 13, still more preferably 7 to 9 carbon atoms (e.g., a monophenylmethyl group, a monophenylpropyl group, and a triphenylmethyl group); aryloxy groups having 5 to 24, preferably 5 to 18, more preferably 5 to 12, still more preferably 5 to 8 carbon atoms (e.g., a phenoxy group, a naphthyloxy group, and a biphenyloxy group); heteroaryl groups having 4 to 24, preferably 4 to 18, more preferably 4 to 12, still more preferably 4 to 8 carbon atoms (e.g., a thiophenyl group, a furanyl group, a carbazole group, a benzothiophenyl group, a benzofuranyl group, an indolyl group, a pyrrolyl group, and a pyridyl group); acyl groups having 1 to 24, preferably 1 to 18, more preferably 1 to 12, still more preferably 1 to 8 carbon atoms (e.g., an acetyl group, a propionyl group, a butanoyl group, a pentanoyl group, a heptanoyl group, and groups obtained by substituting carbonyl groups contained in these acyl groups with ester groups or amide groups); amino groups having 1 to 24, preferably 1 to 18, more preferably 1 to 12, still more preferably 1 to 8 carbon atoms (e.g., a diphenylamino group and a dimethylamino group); fluorine-containing groups such as fluorine and fluorine-containing hydrocarbon groups having 1 to 30, preferably 1 to 12 carbon atoms; a cyano group and a nitro group; and the like.

The substituents may be crosslinked with each other, and the whole substituents may form a cyclic structure (an aromatic group). Each substituent may further have a substituent.

### (X group in formula (I))

Each occurrence of X represents F (fluorine), Cl (chlorine), Br (bromine), or I (iodine).

Of these, Cl (chlorine), Br (bromine), or I (iodine) is preferred in terms of, for example, reactivity and handleability.

The number of X in the compound (I) is not particularly limited as long as it is 1 or greater. The number of X is, for example, 1 to 6, preferably 1 to 4, more preferably 1 to 3.

In the present invention, m, the number of X in the compound (I), is not particularly limited as long as it is an integer and at least one X can react with the metal or metal compound to form an organometallic nucleophile. m is, for example, 1 to 10, preferably 1 to 8, more preferably 1 to 6, still more preferably 1 to 4.

### (Specific examples of compound (I))

Specific examples of A¹ in the compound (I) represented by formula (I) include the following groups.

Naphthyl groups such as a naphthyl group, aryl (e.g., phenyl) naphthyl groups, alkylene-bridged (e.g., ethylene-bridged) naphthyl groups, and arylene-bridged (e.g., phenylene-bridged) naphthyl groups;
a phenanthrenyl group;
anthracenyl groups such as an anthracenyl group, aryl (e.g., phenyl) anthracenyl groups, diaryl (e.g., dinaphthyl) anthracenyl groups, and diarylboryl (e.g., bis(trialkylphenyl)boryl) anthracenyl groups;
pyrenyl groups such as a pyrenyl group and alkyl (e.g., tert-butyl) pyrenyl groups;
biphenyl groups such as a biphenyl group and alkylene-bridged (e.g., propylene-bridged, isopropylene-bridged) biphenyl groups;
polyphenyl groups such as terphenyl groups, tetraaryl (e.g., tetraphenyl) groups, and pentaaryl groups;
a triphenylenyl group;
phenyl groups such as 2-aryl (e.g., phenyl) ethenylphenyl groups, 1,2,2-triaryl (e.g., triphenyl) ethenylphenyl groups, 2-aryl (e.g., phenyl) ethynylphenyl groups, a phenyl group, alkyl (e.g., methyl) phenyl groups, dialkyl (e.g., dimethyl) phenyl groups, alkoxy (e.g., methoxy) phenyl groups, dialkylamino (e.g., dimethylamino) phenyl groups, diaryl (e.g., diphenyl) aminophenyl groups, perfluoroalkyl (e.g., trifluoromethyl) phenyl groups, alkyl (e.g., ethyl) oxycarbonylphenyl groups, and alkanoyl (e.g., acyl) phenyl groups;
aryl-substituted (e.g., phenyl-substituted) carbazolyl groups;
an anthracene-9.10-dione group;
aryl-substituted (e.g., phenyl-substituted) thienyl groups, a thiophene group, and a benzothiadiazole group;
linear or branched alkyl groups having 1 to 20 carbon atoms, such as a methyl group, an ethyl group, a propyl group, an isopropyl group, a n-butyl group, an isobutyl group, a t-butyl group, a n-pentyl group, a n-hexyl group, and a n-octyl group;
cyclic alkyl groups having 3 to 20 carbon atoms, such as a cyclopropyl group, a cyclobutyl group, a cyclopentyl group, and a cyclohexyl group;
unsaturated aliphatic groups having 2 to 20 carbon atoms, such as an ethenyl (vinyl) group, a propenyl (allyl) group, a butenyl group, a pentenyl group, a hexenyl group, an octenyl group, an ethynyl group, a propynyl group, a butynyl group, a pentynyl group, a hexynyl group, and an octynyl group; and
divalent or higher groups obtained by removing one or more hydrogen atoms from these monovalent groups.

Specific examples of the compound (I) used in a cross-coupling reaction method of the present invention include one or more selected from the group consisting of compounds used in Examples 1 to 150.

### <Metal>

The metal or metal compound is not particularly limited as long as it can react with the organohalide to form an organometallic nucleophile. Examples of the metal include one or more selected from the group consisting of alkaline-earth metals, alkali metals, transition metals, zinc, aluminum, indium, tin, bismuth, boron, silicon, gallium, germanium, antimony, lead, and rare-earth metals. Examples of the metal compound include one or more selected from the group consisting of salts of these metals (e.g., chlorides, bromides, iodides, nitrates, sulfates, and carbonates), oxides of these metals, and the like.

Of these, one or more selected from the group consisting of magnesium, calcium, strontium, lithium, manganese, palladium, titanium, zinc, aluminum, bismuth, indium, samarium, salts of these metals, oxides of these metals, and the like are preferred.

The amount of metal used is not particularly limited. Relative to 1 equivalent of the organohalide, the amount of metal is, for example, 0.1 equivalents or more, preferably 0.5 equivalents or more, more preferably 0.7 equivalents or more, and, for example, 10.0 equivalents or less, preferably 5.0 equivalents or less, more preferably 3.0 equivalents or less. If the amount of metal is less than 0.1 equivalents, the reaction may not proceed sufficiently, and the yield may decrease. If the amount of metal is more than 10.0 equivalents, it may be necessary to remove unreacted metal, and the unreacted metal may cause a side reaction.

### <Ether compound>

The ether compound is a compound having one or more ether bonds (-O-) in a molecule, and is not particularly limited as long as it is a compound inactive in the reaction between the organohalide and the metal or metal compound.

Examples include one or more selected from the group consisting of diethyl ether, diisopropyl ether, dibutyl ether, t-butyl methyl ether, tetrahydrofuran, tetrahydropyran, dimethoxyethane, 1,4-dioxane, anisole, acetoxy-2-ethoxyethane, propylene glycol monomethyl ether acetate, ethylene glycol dimethyl ether, diethylene glycol dimethyl ether, 1-methoxy-1,1,2,2-tetrafluoroethane, 1-ethoxy-1,1,2,2-tetrafluoroethane, and the like.

If necessary, the ether compound may be used as a mixture with, for example, an organic solvent.

Examples of the organic solvent include one or more selected from the group consisting of aromatic solvents such as benzene, toluene, xylene, mesitylene durene, and decalin; aliphatic solvents such as hexane, pentane, and heptane; alcohol solvents such as methanol, ethanol, n-propanol, isopropanol, 1-butanol, 1,1-dimethylethanol, tert-butanol, 2-methoxyethanol, and ethylene glycol; halogenated hydrocarbon solvents such as dichloromethane, chloroform, carbon tetrachloride, chlorobenzene, and 1,2-dichlorobenzene; polar solvents such as acetonitrile, N,N'-dimethylformamide, N,N'-dimethylacetamide, N-methyl-2-pyrrolidone, and dimethylsulfoxide; pyridine; and the like.

The amount of ether compound used is 0.5 to 10.0 equivalents relative to 1 equivalent of the organohalide. The amount of ether compound is preferably 0.7 equivalents or more, more preferably 1.0 equivalents or more, still more preferably 1.2 equivalents or more, yet still more preferably 1.5 equivalents or more, and preferably 7.0 equivalents or less, more preferably 5.0 equivalents or less. If the amount of ether compound is less than 0.5 equivalents, the organometallic nucleophile, particularly a Grignard reagent, cannot be efficiently synthesized, and when the organometallic nucleophile is subjected to a subsequent reaction, the reaction may not proceed sufficiently. If the amount of ether compound is more than 10.0 equivalents, since the ether compound functions substantially as a solvent to make it difficult to apply a mechanochemical action to reaction components, the organometallic nucleophile, particularly a Grignard reagent, cannot be efficiently synthesized, and when the organometallic nucleophile is subjected to a subsequent reaction, the reaction may not proceed sufficiently.

### <Mechanochemical process>

The mechanochemical process is a processing method in which mechanical energy is applied to a reactant (particularly, a solid reactant) by means of, for example, shearing, compression, stretching, grinding, rubbing, kneading, mixing, dispersion, disintegration, or shaking to thereby activate the reactant and provide structural change, phase transition, reactivity, adsorbability, catalytic activity, etc. This is expected to lead to surface activation, an increase in surface area, an increase in lattice defect, a decrease in crystal grain size, amorphization, etc.

The device for the mechanochemical process is not particularly limited as long as it is a device capable of applying mechanical energy by the above means.

Examples of such devices include one or more selected from the group consisting of:
mills such as ball mills, rod mills, jet mills, and SAG mills;
grinders such as rotary millstones and grinding machines;
(horizontal-axis rotating) rotary vessel type mixing devices of horizontal cylinder type, V type, double cone type, cube type, S type, continuous V type, etc.;
rotary vessel type mixing devices (with baffle plates and blades) of horizontal cylinder type, V type, double cone type, ball mill type, etc.;
(rotationally oscillating) rotary vessel type mixing devices of rocking type, cross-rotary type, etc.;
(horizontal-axis rotating) fixed vessel type mixing devices of ribbon type, paddle type, single-axis rotor type, bug mill type, etc.;
(vertical-axis rotating) fixed vessel type mixing devices of ribbon type, screw type, planetary type, turbine type, high-speed flow type, rotating disk type, muller type, etc.;
(vibratory) fixed vessel type mixing devices of vibratory mill type, sieve type, etc.;
(fluidal) hydrokinetic mixing devices of heterogeneous fluidized bed type, swirling fluidized bed type, riser tube type, jet pump type, etc.;
(gravitational) hydrokinetic mixing devices of gravity type, static mixer type, etc.;
kneaders such as twin-screw kneaders, single-screw kneaders, mixers, and roll mills;
and the like.

In the present invention, for example, mills such as ball mills, grinders, mixing devices, and kneaders are preferably used.

In the method for producing an organometallic nucleophile according to the present invention, the amount of energy applied when the mechanochemical process is performed is not particularly limited and can be appropriately determined taking into account, for example, the types and amounts of reaction materials and the reaction temperature.

For example, when a ball mill is used, shaking can be performed at 5 Hz or more, preferably 10 Hz or more, more preferably 20 Hz or more.

Although not clear, the principle and other details of the mechanochemical process are probably as follows.

When mechanical energy is applied to raw material compounds, the surfaces of the raw material compounds are activated by absorbing the mechanical energy. As a result, a chemical reaction occurs between the activated surfaces having energy, leading to a reaction between raw material compound molecules. For example, when a ball mill is used, the surfaces of the raw material compounds are abraded and activated (inactive surfaces are removed) upon application of mechanical energy to the raw material compounds.

### <Reaction conditions>

The reaction conditions of the present invention are not particularly limited as long as the organohalide and the metal or metal compound can react with each other to form an organometallic nucleophile.

### (Temperature)

The reaction temperature (the temperature in a reaction vessel during mixing) can be -50°C to 500°C. In the present invention, the reaction can be performed at around room temperature (23°C) without heating. Alternatively, the inside of the reaction vessel (the reaction system) may be heated to a desired temperature by using a heating device such as a heat gun.

The method for controlling the reaction temperature to be -50°C to 500°C is not particularly limited, and a temperature control method used in performing a chemical reaction can be used. Examples of such methods include controlling the temperature in the reaction vessel by immersing the reaction vessel in liquid nitrogen or the like, controlling the temperature in the reaction vessel by using warm air, controlling the temperature in the reaction vessel by covering the reaction vessel with a heating medium at a predetermined temperature, and controlling the temperature in the reaction vessel by providing a heating element.

In the present invention, controlling the temperature in the reaction vessel by applying warm air generated with a heat gun to the reaction vessel is preferred, for example, from the viewpoint of safety and ease of temperature control operation.

### (Pressure)

The pressure is not particularly limited, and the reaction can be performed under any pressure. In this case, a decompression device or a pressurization device can be used. In the present invention, the reaction can be performed without pressurization or decompression.

### (Reaction atmosphere)

The reaction atmosphere (the atmosphere in the reaction vessel during mixing) is not particularly limited and can be appropriately determined taking into account, for example, the types and amounts of organohalide and metal and the reaction temperature.

For example, the reaction can be performed in an air atmosphere without any particular atmosphere adjustment. The reaction can also be performed in an inert gas atmosphere such as nitrogen, helium, neon, or argon.

In general, the synthesis of a Grignard reagent, which is a typical example of an organometallic nucleophile, is performed in an inert atmosphere under conditions avoiding oxygen and water, but the present invention is advantageous in that it is not necessary to create an inert atmosphere and strict control of oxygen and water is not required.

### (Reaction time)

The reaction time (mixing time; the time period during which processing by a mechanical means is performed) is not particularly limited and can be appropriately determined taking into account, for example, the types and amounts of organohalide and metal and the reaction temperature.

For example, the reaction time can be 1 minute or more, preferably 3 minutes or more, more preferably 5 minutes or more. The upper limit of the reaction time is not particularly limited and can be, for example, 10 hours or less, preferably 5 hours or less, more preferably 3 hours or less.

### (Input order of reaction components, treatment after reaction, etc.)

The order in which the components, that is, the organohalide, the metal, and the ether compound, are put in the reaction vessel is not particularly limited. The means of input is also not particularly limited.

After completion of the reaction, the resulting reaction product may be purified as needed. The purification method is not particularly limited, and, for example, a method such as filtration, distillation, recrystallization, column chromatography, or washing with a solvent is used.

### [Reactor]

The reaction vessel provided in a reactor for use in the present invention may be any type of reaction vessel that can be provided in a reactor for compounds, and can be appropriately determined taking into account, for example, the types and amounts of organohalide, metal, ether compound, and reaction product, the reaction temperature, the atmosphere, and the reaction pressure. For example, in the present invention, when a device that mechanically performs mixing treatment (e.g., a ball mill) is used, a ball mill jar or the like can be used as the reaction vessel.

The means for stirring the contents in the reaction vessel provided in the reactor of the present invention may be any type of stirring means that can be provided in a reactor for compounds. In the present invention, means using the devices that mechanically perform mixing treatment described in <Mechanochemical process> above can be used. As the device that mechanically performs mixing treatment, for example, a ball mill is preferably used.

The temperature control means in the reaction vessel provided in the reactor of the present invention is not particularly limited as long as it is a means that controls the temperature so that a cross-coupling reaction is performed at a temperature of -50°C to 500°C. In the present invention, the temperature control means described in (Temperature) in <Reaction conditions> above can be used. For example, it is preferable to heat the reaction vessel by using a heat gun.

The reactor for use in the present invention may further include various means that can be provided in a reactor for compounds, such as a weighing means, a decompression or pressurization means, an atmosphere adjustment means (gas introduction or discharge means), a means of input of components, a means of removal of components and reaction products, a purification means, and an analysis means.

### <Organometallic nucleophile>

Examples of organometallic nucleophiles produced in Embodiment 1 include various ones. Of these, Grignard reagents (organomagnesium chlorides, organomagnesium bromides, and organomagnesium iodides), organocalcium reagents ("Heacy" Grignard reagents), and organomanganese reagents are particularly preferred.

The present inventors measured the near-edge x-ray absorption fine structure (NEXAFS) at the Mg-K absorption edge and the near-edge x-ray absorption fine structure at the C-K absorption edge of PhMgBr prepared mechanochemically in Example 116 and PhMgBr prepared in solution, and compared the spectra with each other.

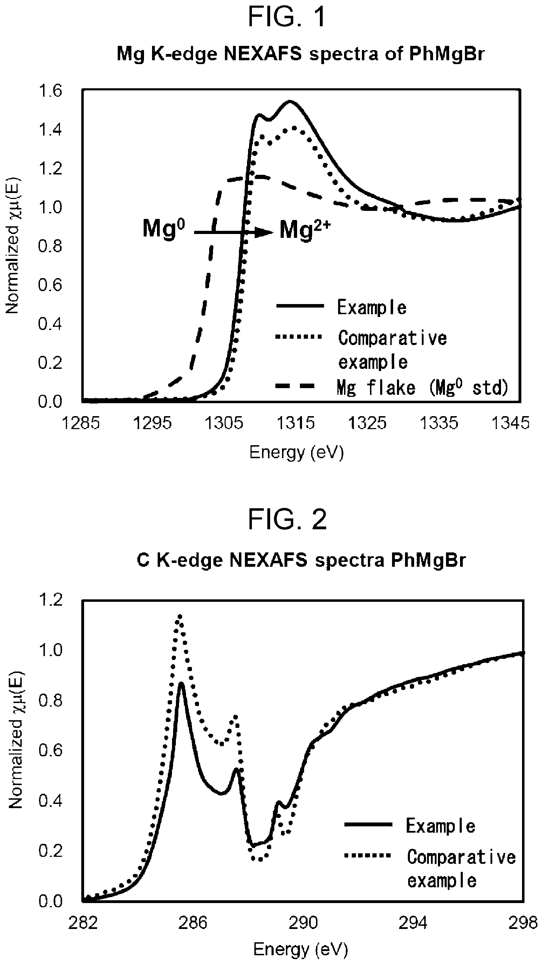

As a result, as shown in Fig. 1 and Fig. 2, the Mg-K and C-K NEXAFS spectra of PhMgBr prepared mechanochemically in Example 116 and PhMgBr prepared in solution are well-approximated, which shows that PhMgBr prepared mechanochemically and PhMgBr prepared in solution are substantially the same organomagnesium species having a carbon-magnesium bond. Furthermore, intense 1s-π* transition peaks are observed at 285.7 eV and 287.7 eV in the C-K NEXAFS spectra, which supports the formation of a carbon-magnesium bond resulting from transformation of a C-Br bond of bromobenzene as a starting material.

### [Embodiment 2]

Embodiment 2 of the present invention is a method for producing an alcohol, including reacting an organohalide and a metal or metal compound with each other by a mechanochemical process in the presence of an ether compound in an amount of 0.5 to 10.0 equivalents relative to 1 equivalent of the organohalide, and further adding an organic carbonyl compound and performing a reaction by the mechanochemical process.

In Embodiment 2, the organohalide, the metal or metal compound, and the ether compound can be the same as those described in Embodiment 1. The conditions of the reaction between the organohalide and the metal or metal compound, and the like can also be the same as those described in Embodiment 1.

Furthermore, in Embodiment 2, the mechanochemical process and the reactor used in the reaction between the organic carbonyl compound and a reaction product of the organohalide and the metal or metal compound can be the same as those described in Embodiment 1.

### <Organic carbonyl compound>

The organic carbonyl compound is not particularly limited as long as it is a compound that has a carbonyl group in a molecule and can undergo a coupling reaction with an organometallic nucleophile (Grignard reagent) to form an alcohol.

Examples include one or more selected from the group consisting of aldehydes, ketones, esters, ketoesters, acetals, and the like.

When an aldehyde is used, a secondary alcohol can be obtained, and when a ketone or an ester is used, a tertiary alcohol can be produced.

The amount of organic carbonyl compound used is not particularly limited. Relative to 1 equivalent of the organohalide, the amount of organic carbonyl compound is, for example, 0.1 equivalents or more, preferably 0.2 equivalents or more, more preferably 0.3 equivalents or more, and, for example, 10.0 equivalents or less, preferably 5.0 equivalents or less, more preferably 3.0 equivalents or less. If the amount of organic carbonyl compound is less than 0.1 equivalents or more than 10.0 equivalents, the reaction may not proceed sufficiently, and the yield may decrease. Furthermore, a component present in excess may cause a side reaction with, for example, the reaction product.

The aldehyde is not particularly limited, and may be, for example, any of aliphatic aldehydes, aromatic aldehydes, heterocyclic aldehydes, and the like. Examples include one or more selected from the group consisting of acetaldehyde, propionaldehyde, butyraldehyde, valeraldehyde, capronaldehyde, cyclohexylaldehyde, isobutyraldehyde, crotonaldehyde, 2-cyclopentenylaldehyde, benzaldehyde, 2-fluorobenzaldehyde, 4-fluorobenzaldehyde, glyoxal, malonaldehyde, succinaldehyde, glutaraldehyde, adipinaldehyde, pimelinaldehyde, sebacinaldehyde, acrolein, salicylaldehyde, phthalaldehyde, furfural, 5-methylfurfural, 2-thiophenecarboxaldehyde, phenylacetaldehyde, o-tolylaldehyde, p-tolylaldehyde, 2,4-dimethylbenzaldehyde, 2-methyl-5-isopropylbenzaldehyde (p-cymene-2-aldehyde), cuminaldehyde, 4-(trifluoromethyl)benzaldehyde, p-anisaldehyde, 2,4-dimethoxybenzaldehyde, 3,4-dimethoxybenzaldehyde (methyl vanillin), 5-methoxy-3,4-methylenedioxybenzaldehyde, 1-naphthoaldehyde, 2-naphthoaldehyde, 6-methoxy-2-naphthoaldehyde, and the like.

Examples of the ketone include, but are not limited to, one or more selected from the group consisting of compounds in which two groups selected from aliphatic hydrocarbon groups, alicyclic hydrocarbon groups, aromatic hydrocarbon groups, and heterocyclic groups are bonded to carbonyl carbon, compounds in which carbonyl carbon constitutes alicyclic hydrocarbon rings, and the like.

Examples include one or more selected from the group consisting of benzophenone, acetone, methyl ethyl ketone, methyl isobutyl ketone, cyclohexanone, acetophenone, propiophenone, acetonaphthone, cyclohexanone, cyclopentanone, 1- or 2-indanone, 1- or 2-tetralone, adamantanone, methyl-2-thienyl ketone, methyl-3-thienyl ketone, methyl-2-pyridyl ketone, 2,2'-dithienyl ketone, diethyl ketone, benzil, acetylacetone, methyl isopropyl ketone, ethyl phenyl ketone, fluorenone, indanone, 3,3,5-trimethylcyclohexanone, anthraquinone, acenaphthenequinone, quinone, benzoylacetone, and the like.

Examples of the ester include, but are not limited to, alkyl esters of aliphatic carboxylic acids and alkyl esters of aromatic carboxylic acids. Examples include one or more selected from the group consisting of ethyl acetate, butyl acetate, ethyl benzoate, butyl benzoate, and the like.

### <Reaction conditions>

In Embodiment 2, the conditions of the reaction between the carbonyl compound and the reaction product of the organohalide and the metal are not particularly limited as long as the carbonyl compound and the reaction product can react with each other to form an alcohol.

### (Temperature, pressure, atmosphere, and time)

The reaction temperature (the temperature in a reaction vessel during mixing) can be -50°C to 500°C. In the present invention, the reaction can be performed at around room temperature (23°C) without heating. Alternatively, the inside of the reaction vessel (the reaction system) may be heated to a desired temperature by using a heating device such as a heat gun.

As the method for controlling the reaction temperature to be -50°C to 500°C, the method described in (Temperature) in <Reaction conditions> of Embodiment 1 can be used.

The pressure is not particularly limited, and the reaction can be performed under any pressure. In this case, a decompression device or a pressurization device can be used. In Embodiment 2 of the present invention, the reaction can be performed without pressurization or decompression.

The reaction atmosphere (the atmosphere in the reaction vessel during mixing) is not particularly limited and can be appropriately determined taking into account, for example, the types and amounts of organometallic nucleophile and carbonyl compound and the reaction temperature.

For example, the reaction can be performed in an air atmosphere without any particular atmosphere adjustment. The reaction can also be performed in an inert gas atmosphere such as nitrogen, helium, neon, or argon. In Embodiment 2 of the present invention, the reaction can be performed in an air atmosphere.

The reaction time (mixing time; the time period during which processing by mechanical means is performed) is not particularly limited and can be appropriately determined taking into account, for example, the types and amounts of organometallic nucleophile and carbonyl compound and the reaction temperature.

For example, the reaction time can be 1 minute or more, preferably 3 minutes or more, more preferably 5 minutes or more. The upper limit of the reaction time is not particularly limited and can be, for example, 10 hours or less, preferably 5 hours or less, more preferably 3 hours or less.

### (Input order of reaction components, treatment after reaction, etc.)

The means for putting the carbonyl compound in the reaction vessel is not particularly limited.

After completion of the reaction, the alcohol obtained as a reaction product can be purified as needed. The purification method is not particularly limited, and, for example, a method such as filtration, distillation, recrystallization, column chromatography, or washing with a solvent can be used.

<Alcohol>

In the method for producing an alcohol according to Embodiment 2, when an aldehyde is used, a secondary alcohol can be obtained, and when a ketone or an ester is used, a tertiary alcohol can be produced.

### [Embodiment 3]

Embodiment 3 of the present invention is a method for producing an alcohol, including reacting an organohalide, a metal or metal compound, and an organic carbonyl compound with each other by a mechanochemical process in the presence of an ether compound in an amount of 0.5 to 10.0 equivalents relative to 1 equivalent of the organohalide.

Embodiment 3 of the present invention is a reaction corresponding to what is called the Barbier reaction.

The organohalide and the ether compound used in Embodiment 3 can be the same as those described in Embodiment 1, and the organic carbonyl compound can be the same as that described in Embodiment 2.

In Embodiment 3, the mechanochemical process and the reactor used in the reaction of the organohalide, the metal, and the organic carbonyl compound can be the same as those described in Embodiment 1.

### <Metal or metal compound>

The metal or metal compound is not particularly limited as long as it can form an alcohol when the organohalide, the metal or metal compound, and the organic carbonyl compound are reacted with each other. Examples include one or more selected from the group consisting of alkaline-earth metals, alkali metals, transition metals, zinc, aluminum, indium, tin, bismuth, boron, silicon, gallium, germanium, antimony, lead, rare-earth metals, salts of these metals (e.g., chlorides, bromides, iodides, nitrates, sulfates, and carbonates), oxides of these metals, and the like.

Of these, one or more selected from the group consisting of magnesium, calcium, strontium, lithium, manganese, palladium, titanium, zinc, aluminum, bismuth, indium, samarium, salts of these metals (e.g., chlorides and iodides), and the like are preferred.

Relative to 1 equivalent of the organohalide, the amount of metal or metal compound used is, for example, 0.1 equivalents or more, preferably 0.5 equivalents or more, more preferably 0.7 equivalents or more, and, for example, 10.0 equivalents or less, preferably 5.0 equivalents or less, more preferably 3.0 equivalents or less. If the amount of metal or metal compound is less than 0.1 equivalents or more than 10.0 equivalents, the reaction may not proceed sufficiently, and the yield may decrease. Furthermore, a component present in excess may cause a side reaction with, for example, the reaction product.

### <Reaction conditions>

In Embodiment 3, the conditions of the reaction between the organohalide, the metal or metal compound, and the organic carbonyl compound are not particularly limited as long as they can react with each other to form an alcohol.

The reaction temperature (the temperature in a reaction vessel during mixing) can be -50°C to 500°C. In the present invention, the reaction can be performed at around room temperature (23°C) without heating. Alternatively, the inside of the reaction vessel (the reaction system) may be heated to a desired temperature by using a heating device such as a heat gun.

As the method for controlling the reaction temperature to be -50°C to 500°C, the method described in (Temperature) in <Reaction conditions> of Embodiment 1 can be used.

The pressure is not particularly limited, and the reaction can be performed under any pressure. In this case, a decompression device or a pressurization device can be used. In Embodiment 3 of the present invention, the reaction can be performed without pressurization or decompression.

The reaction atmosphere (the atmosphere in the reaction vessel during mixing) is not particularly limited and can be appropriately determined taking into account, for example, the types and amounts of organohalide, metal or metal compound, and carbonyl compound and the reaction temperature.

For example, the reaction can be performed in an air atmosphere without any particular atmosphere adjustment. The reaction can also be performed in an inert gas atmosphere such as nitrogen, helium, neon, or argon. In Embodiment 3 of the present invention, the reaction can be performed in an air atmosphere.

The reaction time (mixing time; the time period during which processing by mechanical means is performed) is not particularly limited and can be appropriately determined taking into account, for example, the types and amounts of organohalide, metal or metal compound, and carbonyl compound and the reaction temperature.

For example, the reaction time can be 1 minute or more, preferably 3 minutes or more, more preferably 5 minutes or more. The upper limit of the reaction time is not particularly limited and can be, for example, 10 hours or less, preferably 5 hours or less, more preferably 3 hours or less.

### (Input order of reaction components, treatment after reaction, etc.)

The order in which the components, that is, the organohalide, the metal or metal compound, the ether compound, and the carbonyl compound, are put in the reaction vessel is not particularly limited. The means of input is also not particularly limited.

After completion of the reaction, the resulting reaction product may be purified as needed. The purification method is not particularly limited, and, for example, a method such as filtration, distillation, recrystallization, column chromatography, or washing with a solvent is used.

### [Embodiment 4]

Embodiment 4 of the present invention is an addition reaction method including reacting reaction components including an organohalide, a metal or metal compound, an electrophile, and an ether compound in an amount of 0.5 to 10.0 equivalents relative to 1 equivalent of the organohalide by a mechanochemical process.

In Embodiment 4, Embodiment 4-1 of the present invention relates to an addition reaction method including reacting an organohalide and a metal or metal compound with each other by a mechanochemical process in the presence of an ether compound in an amount of 0.5 to 10.0 equivalents relative to 1 equivalent of the organohalide, and further adding an electrophile and performing a reaction by the mechanochemical process.

In Embodiment 4, Embodiment 4-2 of the present invention relates to an addition reaction method including reacting an organohalide, a metal or metal compound, and an electrophile with each other by a mechanochemical process in the presence of an ether compound in an amount of 0.5 to 10.0 equivalents relative to 1 equivalent of the organohalide.

In Embodiment 4, the organohalide, the metal, and the ether compound can be the same as those described in Embodiment 1. The conditions of the reaction between the organohalide and the metal or metal compound, and the like can also be the same as those described in Embodiment 1.

Furthermore, in Embodiment 4, the mechanochemical process and the reactor used in the reaction between the electrophile and a reaction product of the organohalide and the metal or metal compound can be the same as those described in Embodiment 1.

### <Electrophile>

The electrophile is, for example, one or more selected from the group consisting of compounds having functional groups such as a ketone group, an aldehyde group, a carboxylic acid amide group, a carboxylic acid ester group, a halogen group, a cyano group, a silane halide group, a phosphorus halide (pentavalent) group, a phosphorus halide (trivalent) group, a boron halide group, a group having a boron-oxygen bond, a metal halide group, a heterosilyl group, an imino group, an acyl halide group, an epoxy group, a disulfide group, carbon dioxide (CO₂), a carboxylic anhydride, an imidoyl halide group, and an aziridine group, and is not particularly limited as long as it is a compound that can react with an organometallic nucleophile (Grignard reagent).

Among the electrophiles, as compounds having a ketone group, an aldehyde group, and a carboxylic acid ester group, the carbonyl compounds described in Embodiment 2 can be used.

Among the electrophiles, examples of compounds having a carboxylic acid amide group include compounds obtained by reacting a carboxylic acid such as acetic acid, propionic acid, or benzoic acid with an amine and having a corresponding carboxylic acid amide group.

Among the electrophiles, examples of compounds having a halogen group include the organohalides listed in Embodiment 1, silane compounds having a total of three alkyl and aryl groups and one halogen group, phosphorus compounds (divalent and pentavalent) having a total of two alkyl and aryl groups and one halogen group, boron compounds having a total of three alkyl and aryl groups and one halogen group, and transition metal complexes having a halogen group (e.g., complexes having a structure containing nickel(II) halide, palladium(II) halide, platinum(II) halide, cobalt(I or III) halide, rhodium(I or III) halide, iridium(I or III) halide, copper(I) halide, silver(I) halide, gold(I) halide, iron(II)·(III) halide, ruthenium(II) halide, titanium(IV)·(II) halide, or zirconium(IV) halide and having or not having a ligand).

The amount of electrophile used is not particularly limited. Relative to 1 equivalent of the organohalide, the amount of electrophile is, for example, 0.1 equivalents or more, preferably 0.2 equivalents or more, more preferably 0.3 equivalents or more, and, for example, 10.0 equivalents or less, preferably 5.0 equivalents or less, more preferably 3.0 equivalents or less. If the amount of electrophile is less than 0.1 equivalents or more than 10.0 equivalents, the reaction may not proceed sufficiently, and the yield may decrease. Furthermore, a component present in excess may cause a side reaction with, for example, the reaction product.

### <Reaction conditions>

In Embodiment 4, the conditions of the reaction between the electrophile and the reaction product of the organohalide and the metal are not particularly limited as long as the electrophile and the reaction product can react with each other to form a product.

### (Temperature, pressure, atmosphere, and time)

The reaction temperature (the temperature in a reaction vessel during mixing) can be -50°C to 500°C. In the present invention, the reaction can be performed at around room temperature (23°C) without heating. Alternatively, the inside of the reaction vessel (the reaction system) may be heated to a desired temperature by using a heating device such as a heat gun.

As the method for controlling the reaction temperature to be -50°C to 500°C, the method described in (Temperature) in <Reaction conditions> of Embodiment 1 can be used.

The pressure is not particularly limited, and the reaction can be performed under any pressure. In this case, a decompression device or a pressurization device can be used. In Embodiment 4 of the present invention, the reaction can be performed without pressurization or decompression.

The reaction atmosphere (the atmosphere in the reaction vessel during mixing) is not particularly limited and can be appropriately determined taking into account, for example, the types and amounts of organometallic nucleophile and electrophile and the reaction temperature.

For example, the reaction can be performed in an air atmosphere without any particular atmosphere adjustment. The reaction can also be performed in an inert gas atmosphere such as nitrogen, helium, neon, or argon. In Embodiment 4 of the present invention, the reaction can be performed in an air atmosphere.

The reaction time (mixing time; the time period during which processing by mechanical means is performed) is not particularly limited and can be appropriately determined taking into account, for example, the types and amounts of organometallic nucleophile and electrophile and the reaction temperature.

For example, the reaction time can be 1 minute or more, preferably 3 minutes or more, more preferably 5 minutes or more. The upper limit of the reaction time is not particularly limited and can be, for example, 10 hours or less, preferably 5 hours or less, more preferably 3 hours or less.

### (Input order of reaction components, treatment after reaction, etc.)

The means for putting the electrophile in the reaction vessel is not particularly limited.

After completion of the reaction, the resulting reaction product may be purified as needed. The purification method is not particularly limited, and, for example, a method such as filtration, distillation, recrystallization, column chromatography, or washing with a solvent is used.

### <Reaction product>

In Embodiment 4, various compounds can be obtained depending on the electrophile.

When a compound having a carboxylic acid amide group is used as the electrophile, a corresponding carboxylic acid ester compound can be obtained.

When a compound having a carboxylic acid ester group is used as the electrophile, a corresponding alcohol compound can be obtained.

When a compound having a cyano group is used as the electrophile, a corresponding carboxylic acid ester compound can be obtained.

When a compound having a halogen group is used as the electrophile, a compound in which the halogen group is substituted with an organic group (e.g., an aliphatic hydrocarbon group or an aromatic hydrocarbon group) in the organometallic nucleophile can be obtained.

### [Embodiment 5]

Embodiment 5 of the present invention is a coupling reaction method including reacting reaction components including an organohalide, a metal or metal compound, a sulfonate ester compound, an ether compound in an amount of 0.5 to 10.0 equivalents relative to 1 equivalent of the organohalide, and a nickel catalyst by a mechanochemical process.

In Embodiment 5, Embodiment 5-1 of the present invention is a coupling reaction method including reacting an organohalide and a metal or metal compound with each other by a mechanochemical process in the presence of an ether compound in an amount of 0.5 to 10.0 equivalents relative to 1 equivalent of the organohalide, and further adding a nickel catalyst and a sulfonate ester compound and performing a reaction by the mechanochemical process.

In Embodiment 5, Embodiment 5-2 of the present invention is a coupling reaction method including reacting an organohalide, a metal or metal compound, and a sulfonate ester compound with each other by a mechanochemical process in the presence of a nickel catalyst and an ether compound in an amount of 0.5 to 10.0 equivalents relative to 1 equivalent of the organohalide.

Embodiment 5 of the present invention is a reaction corresponding to what is called the Kumada-Tamao-Corriu coupling reaction.

In Embodiment 5, the organohalide, the metal, and the ether compound can be the same as those described in Embodiment 1. The conditions of the reaction between the organohalide and the metal or metal compound, and the like can also be the same as those described in Embodiment 1.

Furthermore, in Embodiment 5, the mechanochemical process and the reactor used in the reaction between the sulfonate ester compound and a reaction product of the organohalide and the metal or metal compound in the presence of the nickel catalyst can be the same as those described in Embodiment 1.

### <Nickel catalyst>

The nickel catalyst is not particularly limited as long as it can catalyze the reaction between an organometallic nucleophile and the sulfonate ester compound. Examples include one or more selected from the group consisting of nickel(II) chloride, nickel(II) bromide, nickel(II) iodide, nickel(II) nitrate, nickel(II) acetate, nickel(II) sulfate, nickel(II) carbonate, nickel formate, nickel acetate, nickel(II) acetylacetonate, dichloro[bis(triphenylphosphine)]nickel(II) [Ni(PPh₃)2Cl₂], dibromo[bis(triphenylphosphine)]nickel(II) [Ni(PPh₃)2Br₂], dichloro[bis(diphenylphosphino)methane]nickel(II) [Ni(dppm)Cl₂], dichloro [1,2-bis(diphenylphosphino)ethane]nickel(II) [Ni(dppe)Cl₂], dichloro[1,3-bis(diphenylphosphino)propane]nickel(II) [Ni(dppp)Cl₂], dichloro(ethylenediamine)nickel, dichloro(N,N,N',N'-tetramethylethylenediamine)nickel(II), dichloro(N,N,N',N'-tetramethylethylenediamine)nickel(II)/triphenylphosphine mixtures, dichloro[bis(tri-n-butylphosphine)]nickel(II), [1,1'-bis(diphenylphosphino)iron]nickel(II) dichloride, bis(1,5-cyclooctadiene)nickel(0), bis(1,5-cyclooctadiene)nickel(0)/triphenylphosphine mixtures, nickel(0) carbonyl, tetrakis(triphenylphosphine)nickel(0), and the like.

The amount of nickel catalyst used is not particularly limited. Relative to 1 equivalent of the organohalide, the amount of nickel catalyst is, for example, 1.0 × 10⁻³ equivalents or more, preferably 0.01 equivalents or more, more preferably 0.1 equivalents or more, and, for example, 3.0 equivalents or less, preferably 2.0 equivalents or less, more preferably 1.0 equivalents or less, still more preferably 0.5 equivalents or less. If the amount of nickel catalyst is less than 1.0 × 10⁻³ equivalents or more than 3.0 equivalents, the reaction may not proceed smoothly.

### <Sulfonate ester compound>

The sulfonate ester compound is not particularly limited as long as it undergoes a coupling reaction (Kumada-Tamao-Corriu coupling reaction) with the organometallic nucleophile in the presence of the nickel catalyst.

Examples include one or more selected from the group consisting of C1 to C10 alkyl esters, C6 to C20 aryl esters, C6 to C20 heteroaryl esters, C3 to C10 cycloalkyl esters, and the like of arenesulfonic acids such as p-toluenesulfonic acid, benzenesulfonic acid, and naphthalenesulfonic acid.

The amount of sulfonate ester compound used is not particularly limited. Relative to 1 equivalent of the organohalide, the amount of sulfonate ester compound is, for example, 0.1 equivalents or more, preferably 0.2 equivalents or more, more preferably 0.3 equivalents or more, and, for example, 10.0 equivalents or less, preferably 5.0 equivalents or less, more preferably 3.0 equivalents or less. If the amount of sulfonate ester compound is less than 0.1 equivalents or more than 10.0 equivalents, the reaction may not proceed sufficiently, and the yield may decrease. Furthermore, a component present in excess may cause a side reaction with, for example, the reaction product.

### <Reaction conditions>

In Embodiment 5, the conditions of the reaction between the sulfonate ester compound and the reaction product of the organohalide and the metal in the presence of the nickel catalyst are not particularly limited as long as the sulfonate ester compound and the reaction product can react with each other to form a product.

### (Temperature, pressure, atmosphere, and time)

The reaction temperature (the temperature in a reaction vessel during mixing) can be -50°C to 500°C. In the present invention, the reaction can be performed at around room temperature (23°C) without heating. Alternatively, the inside of the reaction vessel (the reaction system) may be heated to a desired temperature by using a heating device such as a heat gun.

As the method for controlling the reaction temperature to be -50°C to 500°C, the method described in (Temperature) in <Reaction conditions> of Embodiment 1 can be used.

The pressure is not particularly limited, and the reaction can be performed under any pressure. In this case, a decompression device or a pressurization device can be used. In Embodiment 5 of the present invention, the reaction can be performed without pressurization or decompression.

The reaction atmosphere (the atmosphere in the reaction vessel during mixing) is not particularly limited and can be appropriately determined taking into account, for example, the types and amounts of organometallic nucleophile and sulfonate ester compound and the reaction temperature.

For example, the reaction can be performed in an air atmosphere without any particular atmosphere adjustment. The reaction can also be performed in an inert gas atmosphere such as nitrogen, helium, neon, or argon. In Embodiment 5 of the present invention, the reaction can be performed in an air atmosphere.

The reaction time (mixing time; the time period during which processing by mechanical means is performed) is not particularly limited and can be appropriately determined taking into account, for example, the types and amounts of organometallic nucleophile and sulfonate ester compound and the reaction temperature.

For example, the reaction time can be 1 minute or more, preferably 3 minutes or more, more preferably 5 minutes or more. The upper limit of the reaction time is not particularly limited and can be, for example, 10 hours or less, preferably 5 hours or less, more preferably 3 hours or less.

### (Input order of reaction components, treatment after reaction, etc.)

The means for putting the electrophile in the reaction vessel is not particularly limited.

After completion of the reaction, the resulting reaction product may be purified as needed. The purification method is not particularly limited, and, for example, a method such as filtration, distillation, recrystallization, column chromatography, or washing with a solvent is used.

### <Reaction product>

In Embodiment 5, various coupling reaction products can be obtained depending on the organohalide and the sulfonate ester compound. Specifically, a coupling reaction product in which a halogen group in the organohalide is substituted with an ester group in the sulfonate ester compound can be obtained.

### [Embodiment 6]

Embodiment 6 of the present invention is an addition reaction method including reacting reaction components including an organohalide, a metal or metal compound, a conjugated enone compound, and an ether compound in an amount of 0.5 to 10.0 equivalents relative to 1 equivalent of the organohalide by a mechanochemical process.

In Embodiment 6, Embodiment 6-1 of the present invention is an addition reaction method including reacting an organohalide and a metal or metal compound with each other by a mechanochemical process in the presence of an ether compound in an amount of 0.5 to 10.0 equivalents relative to 1 equivalent of the organohalide, and further adding a conjugated enone compound and performing a reaction by the mechanochemical process.

In Embodiment 6, Embodiment 6-2 of the present invention is an addition reaction method including reacting an organohalide, a metal or metal compound, and a conjugated enone compound with each other by a mechanochemical process in the presence of an ether compound in an amount of 0.5 to 10.0 equivalents relative to 1 equivalent of the organohalide.

In Embodiment 6, the organohalide, the metal or metal compound, and the ether compound can be the same as those described in Embodiment 1. The conditions of the reaction between the organohalide and the metal or metal compound, and the like can also be the same as those described in Embodiment 1.

Furthermore, in Embodiment 6, the mechanochemical process and the reactor used in the reaction between the conjugated enone compound and a reaction product of the organohalide and the metal or metal compound can be the same as those described in Embodiment 1.

<Conjugated enone compound>

Examples of the conjugated enone compound include compounds having a -CO-CH=CH- structure in a molecule. The two ends of the enone structure (-CO-CH=CH-) may be, for example, one or more selected from the group consisting of hydrogen, an aromatic hydrocarbon group, an aliphatic hydrocarbon group, and a heterocyclic group, and groups at the two ends may be bonded to each other to form a ring.

Examples of the conjugated enone compound include one or more selected from the group consisting of methyl vinyl ketone, 2-cyclohexen-1-one, 2-cyclopenten-1-one, 2-cyclohepten-1-one, 1-phenyl-2-buten-1-one, 5-methyl-3-hexen-2-one, 3-nonen-2-one, benzylideneacetone, 5,6-dihydro-2H-pyran-2-one, 3-buten-2-one, 3-nonen-2-one, 1,3-diphenyl-2-propen-1-one, 3-phenyl-2-propenal, acrylaldehyde, and the like.

The amount of conjugated enone compound used is not particularly limited. Relative to 1 equivalent of the organohalide, the amount of conjugated enone compound is, for example, 0.1 equivalents or more, preferably 0.2 equivalents or more, more preferably 0.3 equivalents or more, and, for example, 10.0 equivalents or less, preferably 5.0 equivalents or less, more preferably 3.0 equivalents or less. If the amount of conjugated enone compound is less than 0.1 equivalents or more than 10.0 equivalents, the reaction may not proceed sufficiently, and the yield may decrease. Furthermore, a component present in excess may cause a side reaction with, for example, the reaction product.

### [Embodiment 7]

Embodiment 7 of the present invention is a composition obtained by the method including reacting an organohalide and a metal or metal compound with each other by a mechanochemical process in the presence of an ether compound in an amount of 0.5 to 10.0 equivalents relative to 1 equivalent of the organohalide. The composition contains a halogenated organometallic compound and an ether compound, and a content of the ether compound is 0.5 to 10 equivalents relative to 1 equivalent of the halogenated organometallic compound.

The organohalide, the metal or metal compound, and the ether compound used in Embodiment 7 may be, for example, the same as those described in Embodiment 1. The mechanochemical process and the reactor used in the reaction can also be the same as those described in Embodiment 1.

The composition according to Embodiment 7 is obtained, for example, as a muddy mixture (slurry) by the method including reacting an organohalide and a metal or metal compound with each other by a mechanochemical process in the presence of an ether compound in an amount of 0.5 to 10.0 equivalents relative to 1 equivalent of the organohalide.

Conventionally, organometallic nucleophiles such as Grignard reagents are synthesized after reaction materials are dissolved in a solvent and contain a large amount of organic solvent, and thus, organometallic nucleophiles with low organic solvent content have hitherto been uncommon. The composition according to Embodiment 7 of the present invention includes a slurry-like composition with low solvent content and is advantageous in terms of, for example, handleability and reaction efficiency.

### [Embodiment 8]

Embodiment 8 of the present invention is a halogenated organometallic compound-ether compound complex including at least a halogenated organometallic compound and an ether compound. The complex is represented by formula (A):

[R¹¹-(MX)ₚ]_{q}-rE (A)

(where R¹¹ is a p-valent organic group derived from the halogenated organometallic compound, M is a metal derived from the halogenated organometallic compound, X is a halogen, and E is the ether compound; a plurality of R^{11'}s, M's, X's, or E's, if present, may be the same or different; and p is an integer of 1 or greater, q is a number of 2 or greater, and r is a number greater than 0).

A¹¹ in formula (A) represents the same group as A¹ in formula (I) relating to the organohalide described in Embodiment 1, that is, an optionally substituted m-valent aromatic hydrocarbon group, an optionally substituted m-valent aromatic heterocyclic group, an optionally substituted m-valent aliphatic hydrocarbon group, or an optionally substituted m-valent unsaturated aliphatic hydrocarbon group. Specific examples of the group are the same as those described in Embodiment 1.

M in formula (A) is the same as the metal or the metal constituting the metal compound described in Embodiment 1.

Each occurrence of X in formula (A) represents F (fluorine), Cl (chlorine), Br (bromine), or I (iodine).

E in formula (A) is an ether compound coordinated to the halogenated organometallic compound and may be, for example, the ether compound described in Embodiment 1.

p in formula (A) represents the number of halogenated metal groups in the halogenated organometallic compound, and is an integer of 1 or greater, preferably an integer of 1 to 6, more preferably an integer of 1 to 3.

q in formula (A) is the quantity of halogenated organometallic compounds constituting the complex, and r in formula (A) is the quantity of ether compounds constituting the complex (the number of ether compounds coordinated to the halogenated organometallic compound).

q is, for example, 1 or greater, preferably 2 or greater, more preferably 2 to 12.

r is a number greater than 0, for example, 0.5 or greater, and is preferably 1 or greater, more preferably an integer of 2 to 12.

The halogenated organometallic compound-ether compound complex represented by formula (A):

[R¹¹-(MX)ₚ]_{q}-rE (A)

(where R¹¹ is a p-valent organic group derived from the halogenated organometallic compound, M is a metal derived from the halogenated organometallic compound, X is a halogen, and E is the ether compound; a plurality of R^{11'}s, M's, X's, or E's, if present, may be the same or different; and p is an integer of 1 or greater, q is a number of 2 or greater, and r is a number greater than 0) can be obtained by the method for producing an organometallic nucleophile according to Embodiment 1. The halogenated organometallic compound-ether compound complex can be easily obtained also by using other methods.

For example, in Example 116, an ether compound (tetrahydrofuran; THF) in an amount of 2.0 equivalents relative to 1 equivalent of an organohalide (bromobenzene) is added in the reaction system.

It is known that in a Grignard reagent in solution, solvent molecules coordinate to Mg atoms. To determine how the ether compound (THF) affects the structure of a Grignard reagent obtained mechanochemically by solid-phase reaction, solid structure analysis was performed using model complexes [Ph-MgBr]₄-rTHF (r = 0, 2, 4, 6, and 8) through a comparison between theoretical calculations and NEXAFS spectra.

To determine the most stable structures of these complexes, using a quantum chemical calculation program "ORCA", minimum-only sampling calculations were conducted using the SC-AFIR method at the GFN-xTB level of theory, where the model collision energy parameter Gamma of the AFIR method was set at 200 kJ/mol.

As a result, 1623 locally stable structures were obtained for [Ph-MgBr]₄. Next, the most stable structure was selected, and 2, 4, 6, or 8 THF molecules were added to perform the second round of sampling calculations. In the case of [Ph-MgBr]₄-rTHF, 1511, 1426, 948, and 750 locally stable structures were eventually provided for r = 2, 4, 6, and 8, respectively. The stable structures obtained by the sampling calculations were subsequently re-optimized by theoretical calculations at the B3LYP-D3/Def2SVP level using the RIJCOSX approximation (implemented in the ORCA package). In the RIJCOSX approximation, an auxiliary basis set called def2/J was used. Frequency calculations were also performed at the same level of theory to confirm that all the optimized structures were minima. Since this reaction was performed in the solid state, no solvation model is adopted. All Gibbs energies were evaluated at 298.15 K and 1 atm.

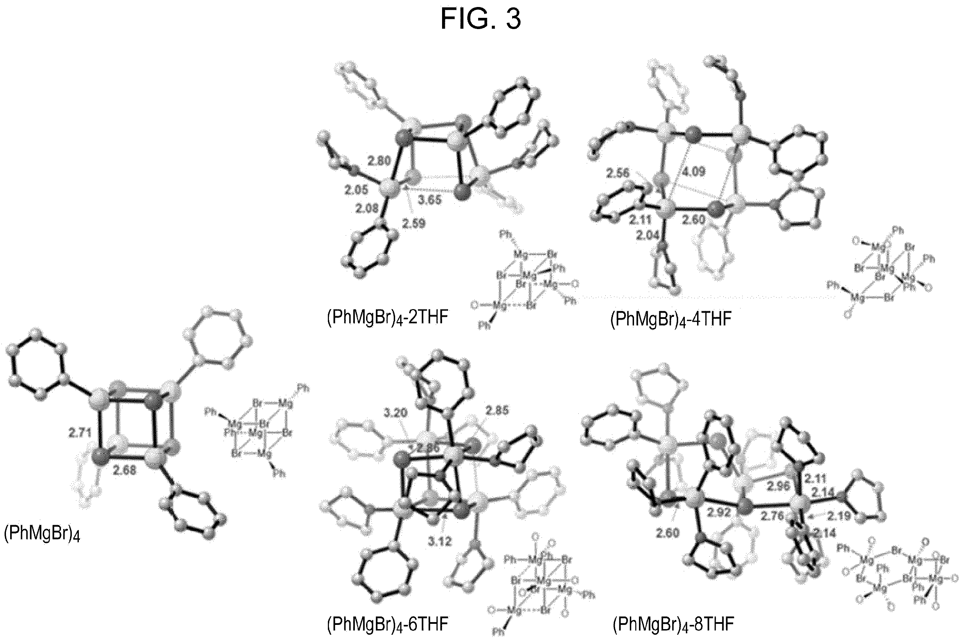

Fig. 3 shows optimized structures of halogenated organometallic compound-ether compound complexes represented by [Ph-MgBr]₄-rTHF at the B3LYP-D3/Def2SVP level, where r = 0, 2, 4, 6, and 8. All hydrogen atoms are omitted for clarity.

As shown in Fig. 3, the most stable [PhMgBr]₄ isomer was found to have a cubic structure. The THF molecules were additionally coordinated to the Mg atoms, and this coordination eventually induced Mg-Br bond scission in the cubic structure. This shows that the coordination of the THF molecules transformed the cubic structure into more open structures.

That is, Fig. 3 shows that when THF (ether compound) is contained in a complex, the cubic structure of a halogenated organometallic compound not containing THF (r = 0) transforms into a more open structure, and coordination of THF to Mg atoms accompanied by Mg-Br bond scission occurs.

Therefore, it is presumed from Fig. 1 to Fig. 3 that the compound represented by formula (A):

[R¹¹-(MX)ₚ]_{q}-rE (A)

is transformed from a less reactive closed form into a more reactive open form under the influence of the coordinating ether compound. Note that the present invention is not bound by this presumption.

### [Embodiment 9]

Embodiment 9 of the present invention is an organometallic nucleophile obtained by reacting an organohalide having a solubility at 20°C in an ether compound of less than 1.0 mol/L and a metal or metal compound with each other by a mechanochemical process in the presence of an ether compound in an amount of 0.5 to 10.0 equivalents relative to 1 equivalent of the organohalide.

The organohalide having a solubility at 20°C in an ether compound of less than 1.0 mol/L is not particularly limited. For example, among the organohalides described in Embodiment 1, organohalides having a solubility at 20°C in an ether compound of less than 1.0 mol/L may be used. In particular, a compound represented by formula (II):

A²-(A³)ₙ-A⁴-X (II)

(where A² is an optionally substituted monovalent aromatic group, an optionally substituted monovalent heterocyclic group, or an optionally substituted monovalent cycloaliphatic group; A³ and A⁴ are each an optionally substituted monovalent aromatic group, an optionally substituted monovalent heterocyclic group, or an optionally substituted monovalent cycloaliphatic group; a plurality of A³'s, if present, may be the same or different from each other, and A³ and A⁴ may be the same or different from each other; and n is an integer of 1 to 10) may be used. Examples of rings constituting A², A³, and A⁴ include a benzene ring, a naphthalene ring, a cyclohexane ring, a thiophene ring, a pyrrole ring, a furan ring, an oxadiazole ring, and a pyridine ring. The compound is, for example, a compound (4-bromo-p-terphenyl) used in Example 115.

The organohalide having a solubility at 20°C in an ether compound of less than 1.0 mol/L does not sufficiently dissolve in an organic solvent such as an ether solvent, and thus is a compound that has not been able to be a raw material for an organometallic nucleophile such as a Grignard reagent.

Embodiment 9 of the present invention is an organometallic nucleophile based on a compound that could not previously be used as a raw material for an organometallic nucleophile such as a Grignard reagent, and is extremely useful as a reagent that expands the possibilities of organic synthesis.

Examples of the metal or metal compound and the ether compound used in Embodiment 9 include those described in Embodiment 1.

In Embodiment 9, the mechanochemical process and the reactor used in the reaction between the organohalide and the metal or metal compound can be the same as those described in Embodiment 1.

### [Other usable components]

In Embodiments 1 to 9 of the present invention, in addition to the organohalide, the metal or metal compound, the ether compound, the organic carbonyl compound, the electrophile, the nickel catalyst, the sulfonate ester compound, and the conjugated enone compound, components that have previously been used in the synthesis of an organometallic nucleophile (Grignard reagent) and components used in the reaction between the organometallic nucleophile (Grignard reagent) and the organic carbonyl compound, the electrophile, the nickel catalyst, the sulfonate ester compound, or the conjugated enone compound can be used as other components.

### EXAMPLES

The present invention will now be described in more detail with reference to specific examples. Note that these specific examples are given by way of illustration only and should not be construed as limiting the present invention.

Unless otherwise specified, compounds used in the specific examples were commercially available products, which were directly used without purification.

In structural formulae of the compounds used in the examples, Me represents a methyl group, t-Bu or tBu represents a tertiary butyl group, Ph represents a phenyl group, Ts represents a tosyl group (p-toluenesulfonyl group), and TBS represents a tert-butyldimethylsilyl group.

"Equiv" denotes "equivalent".

In Examples and Comparative Examples, when a reaction was performed using a ball mill, reagents were put in a stainless-steel ball mill jar, and a ball mill of MM400 model manufactured by Verder Scientific Co., Ltd. (formerly Retsch) was used.

When a cross-coupling reaction was performed using a ball mill, heating was performed such that the outside of a ball mill jar was heated at a predetermined temperature with a heat gun (HG-1450B manufactured by Takagi Co., Ltd.).

The relationship between the preset temperature of the heat gun and the temperature inside the ball mill jar (the temperature in the reaction system) was determined using a thermographic image and found to be as follows.

**[Table 1]**

| Heat gun temperature | Temperature inside jar |
|---|---|
| 250°C | 119.9°C |
| 200°C | 99.5°C |
| 150°C | 74.0°C |
| No heating | 32.1°C |

### [Examples 1 to 60]

### <Method for producing alcohol, including reacting organohalide and metal or metal compound with each other by mechanochemical process in presence of ether compound in amount of 0.5 to 10.0 equivalents relative to 1 equivalent of organohalide, and further adding organic carbonyl compound and performing reaction by mechanochemical process>

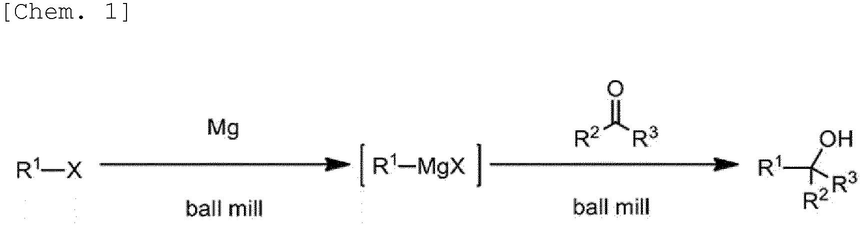

### [Example 1]

To a 5 mL stainless-steel ball mill jar containing stainless-steel balls 10 mm in diameter, 128.3 mg (0.75 mmol, 1.5 equiv) of 4-bromotoluene as an organohalide, 18.2 mg (0.75 mmol, 1.5 equiv) of magnesium as a metal, and 123 µL (1.5 mmol, 3.0 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped, and 53.1 mg (0.5 mmol, 1.0 equiv) of benzaldehyde as an organic carbonyl compound was added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be 91%. The crude mixture was purified by silica gel column chromatography to isolate a target alcohol ((4-methylphenyl)phenylmethanol) (86.1 mg, 0.43 mmol, isolated yield 86%).

### [Examples 2 to 30]

The reaction was performed in the same manner as in Example 1 to obtain reaction products, except that compounds in Table 2 were used as organohalides and organic carbonyl compounds and the amounts of the components used were as follows: organohalide, 1.5 equiv; metal (magnesium), 1.5 equiv; ether compound (tetrahydrofuran), 3.0 equiv; and organic carbonyl compound, 0.05 mmol (1.0 equiv). The results are shown in Table 2 together with isolated yields and NMR yields.

**[Table 2]**

| Example | Organohalide | Organic carbonyl compound | Reaction product | Isolated yield (%) | NMR yield (%) |
|---|---|---|---|---|---|
| 1 | 11a | 12a | 13aa | 86 | 91 |
| 2 | 11a | 12b | 13ab | 70 | 86 |
| 3 | 11a | 12c | 13ac | 62 | 66 |
| 4 | 11a | 12d | 13ad | 74 | 80 |
| 5 | 11a | 12e | 13ae | 69 | 72 |
| 6 | 11a | 12f | 13af | 72 | 81 |
| 7 | 11b | 12a | 13ba | 84 | 94 |
| 8 | 11b | 12b | 13bb | 70 | 84 |
| 9 | 11b | 12c | 13bc | 73 | 75 |
| 10 | 11b | 12d | 13bd | 82 | 88 |
| 11 | 11b | 12e | 13be | 79 | 83 |
| 12 | 11b | 12f | 13bf | 73 | 83 |
| 13 | 11c | 12a | 13ca | 77 | 81 |
| 14 | 11c | 12b | 13cb | 70 | 81 |
| 15 | 11c | 12c | 13cc | 62 | 70 |
| 16 | 11c | 12d | 13cd | 71 | 71 |
| 17 | 11c | 12e | 13ce | | 54 |
| 18 | 11c | 12f | 13cf | | 48 |
| 19 | 11d | 12a | 13da | 73 | 90 |
| 20 | 11d | 12b | 13db | 46 | 62 |
| 21 | 11d | 12c | 13dc | 67 | 70 |
| 22 | 11d | 12d | 13dd | | 58 |
| 23 | 11d | 12e | 13de | | 66 |
| 24 | 11d | 12f | 13df | 68 | 73 |
| 25 | 11e | 12a | 13ea | | 24 |
| 26 | 11e | 12b | 13eb | 40 | |
| 27 | 11e | 12c | 13ec | | 26 |
| 28 | 11e | 12d | 13ed | | 33 |
| 29 | 11e | 12e | 13ee | 15 | |
| 30 | 11e | 12f | 13ef | | 19 |

### [Examples 31 to 60]

The reaction was performed in the same manner as in Example 1 to obtain reaction products, except that compounds in Table 3 were used as organohalides and organic carbonyl compounds and the amounts of the components used were as follows: organohalide, 2.0 equiv; metal (magnesium), 5.0 equiv; ether compound (tetrahydrofuran), 3.0 equiv; and organic carbonyl compound, 0.05 mmol (1.0 equiv). The results are shown in Table 3 together with isolated yields and NMR yields.

**[Table 3]**

| Example | Organohalide | Organic carbonyl compound | Reaction product | Isolated yield (%) | NMR yield (%) |
|---|---|---|---|---|---|
| 31 | 11a | 12a | 13aa | 56 | 78 |
| 32 | 11a | 12b | 13ab | 42 | 61 |
| 33 | 11a | 12c | 13ac | 55 | 68 |
| 34 | 11a | 12d | 13ad | 64 | 70 |
| 35 | 11a | 12e | 13ae | 60 | 85 |
| 36 | 11a | 12f | 13af | 68 | 75 |
| 37 | 11b | 12a | 13ba | 64 | 75 |
| 38 | 11b | 12b | 13bb | 63 | |
| 39 | 11b | 12c | 13bc | 58 | 67 |
| 40 | 11b | 12d | 13bd | 45 | 63 |
| 41 | 11b | 12e | 13be | 69 | 82 |
| 42 | 11b | 12f | 13bf | 73 | 82 |
| 43 | 11c | 12a | 13ca | 67 | 75 |
| 44 | 11c | 12b | 13cb | 61 | |
| 45 | 11c | 12c | 13cc | 55 | 62 |
| 46 | 11c | 12d | 13cd | 61 | 74 |
| 47 | 11c | 12e | 13ce | 72 | |
| 48 | 11c | 12f | 13cf | 65 | 72 |
| 49 | 11d | 12a | 13da | 77 | 92 |
| 50 | 11d | 12b | 13db | 62 | |
| 51 | 11d | 12c | 13dc | 55 | 67 |
| 52 | 11d | 12d | 13dd | 60 | 71 |
| 53 | 11d | 12e | 13de | 63 | 77 |
| 54 | 11d | 12f | 13df | 99 | |
| 55 | 11e | 12a | 13ea | 58 | 74 |
| 56 | 11e | 12b | 13eb | 42 | 50 |
| 57 | 11e | 12c | 13ec | 43 | 44 |
| 58 | 11e | 12d | 13ed | 30 | 41 |
| 59 | 11e | 12e | 13ee | 22 | 38 |
| 60 | 11e | 12f | 13ef | 50 | 51 |

The organohalides in Table 2 and Table 3 are as shown below.

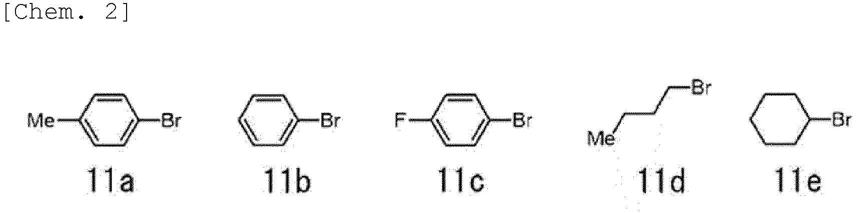

The organic carbonyl compounds in Table 2 and Table 3 are as shown below.

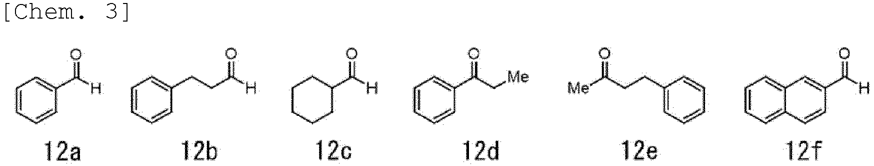

The reaction products in Table 2 and Table 3 are as shown below.

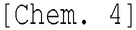

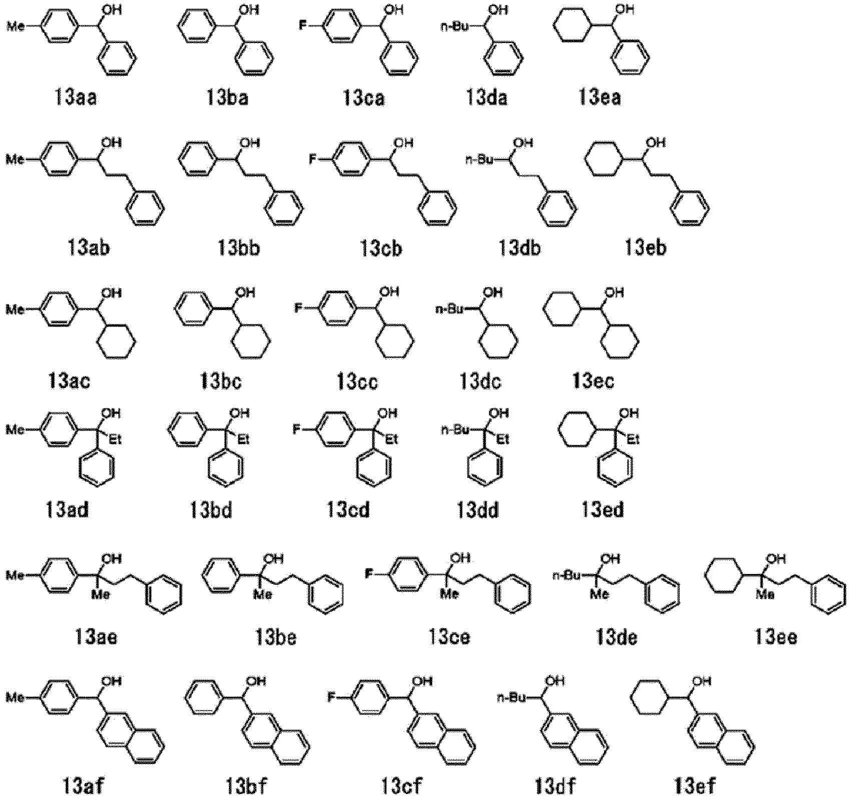

### [Example 61]

To a 5 mL stainless-steel ball mill jar containing stainless-steel balls 10 mm in diameter, 207.1 mg (1.00 mmol, 2.0 equiv) of 2-bromonaphthalene as an organohalide, 60.8 mg (2.50 mmol, 5.0 equiv) of magnesium as a metal, and 123 µL (1.5 mmol, 3.0 equiv) of tetrahydrofuran as an ether compound were added under air. The ball mill jar was capped, mounted to a ball mill, and stirred by shaking (30 Hz) for 60 minutes while being heated at a heat gun temperature of 110°C to cause a reaction. Thereafter, the ball mill jar was uncapped, and 53.1 mg (0.5 mmol, 1.0 equiv) of benzaldehyde as an organic carbonyl compound and 205 µL (5.0 equiv) of tetrahydrofuran as an ether compound were added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be 85%. The crude mixture was purified by silica gel column chromatography to isolate a target alcohol (phenyl(2-naphthyl)methanol). The isolated yield was 77%.

### [Examples 62 to 66]

The reaction was performed in the same manner as in Example 61 to obtain reaction products, except that compounds in Table 4 were used as organohalides and organic carbonyl compounds and the amounts of the components used were as follows: organohalide, 2.0 equiv; metal, 5.0 equiv; ether compound, 3.0 equiv; and organic carbonyl compound, 0.50 mmol (1.0 equiv). The results are shown in Table 4 together with isolated yields.

### [Examples 67 to 72]

The reaction was performed in the same manner as in Example 61 to obtain reaction products, except that compounds in Table 4 were used as organohalides and organic carbonyl compounds and the amounts of the components used were as follows: organohalide, 1.5 equiv; metal (magnesium), 1.5 equiv; ether compound (tetrahydrofuran), 3.0 equiv; and organic carbonyl compound, 0.50 mmol (1.0 equiv). The results are shown in Table 4 together with isolated yields and NMR yields.

**[Table 4]**

| Example | Organohalide | Organic carbonyl compound | Reaction product | Isolated yield (%) | NMR yield (%) |
|---|---|---|---|---|---|
| 61 | 21a | 22a | 23aa | 77 | |
| 62 | 21b | 22a | 23ba | 75 | 93 |
| 63 | 21b | 22b | 23bb | 76 | 81 |
| 64 | 21c | 22a | 23ca | 82 | |
| 65 | 21d | 22a | 23da | 79 | |
| 66 | 21e | 22a | 23ea | 75 | |
| 67 | 21a | 22a | 23aa | | 61 |
| 68 | 21b | 22a | 23ba | 76 | 80 |
| 69 | 21b | 22b | 23bb | 60 | 72 |
| 70 | 21c | 22a | 23ca | | 61 |
| 71 | 21d | 22a | 23da | <1 | |
| 72 | 21e | 22a | 23ea | | 35 |

The organohalides in Table 4 are as shown below.

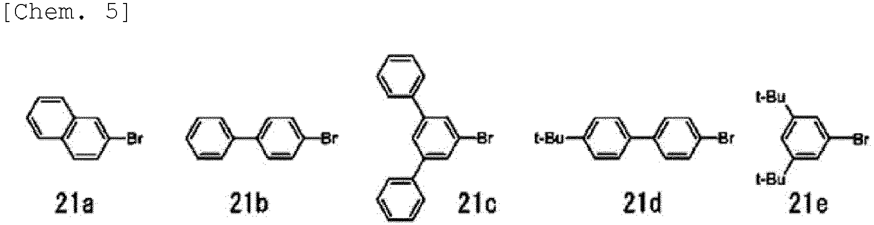

The organic carbonyl compounds in Table 4 are as shown below.

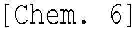

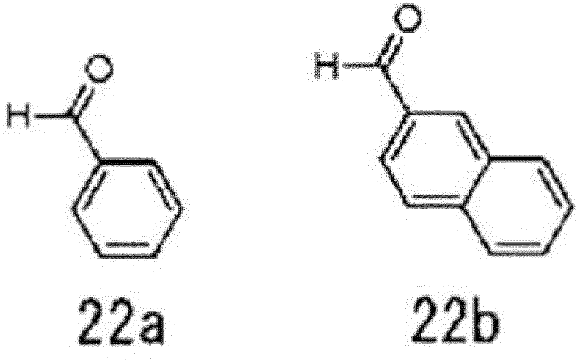

The reaction products in Table 4 are as shown below.

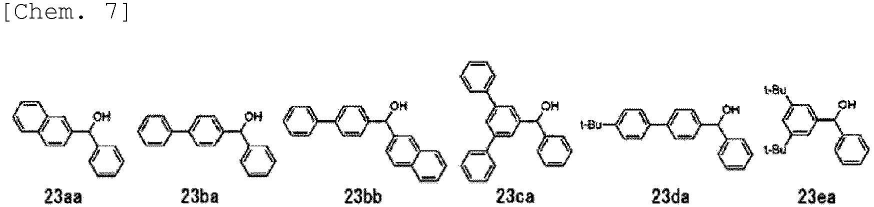

### [Examples 73 to 77 and Comparative Examples 1 and 2]

### <Technical significance of ether compound>

### [Example 73]

To a 5 mL stainless-steel ball mill jar containing stainless-steel balls 10 mm in diameter, 128.3 mg (0.75 mmol, 1.5 equiv) of 4-bromotoluene as an organohalide, 18.2 mg (0.75 mmol, 1.5 equiv) of magnesium as a metal, and 123 µL (1.5 mmol, 3.0 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped to check the inside, and as a result, it was found that a muddy mixture (slurry) with light orange color was produced.

Thereafter, 53.1 mg (0.5 mmol, 1.0 equiv) of benzaldehyde as an organic carbonyl compound was further added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. The dichloromethane was removed with an evaporator to obtain a reaction product (diphenylmethanol). The NMR yield was determined by 1H NMR to be 91%.

Thus, a composition containing a halogenated organomagnesium compound and an ether compound, the content of the ether compound being 0.5 to 10 equivalents relative to 1 equivalent of the halogenated organomagnesium compound, was obtained.

### [Comparative Example 1]

To a 5 mL stainless-steel ball mill jar containing stainless-steel balls 10 mm in diameter, 128.3 mg (0.75 mmol, 1.5 equiv) of 4-bromotoluene as an organohalide and 18.2 mg (0.75 mmol, 1.5 equiv) of magnesium as a metal were added under air. The ball mill jar was capped, mounted to a ball mill, and stirred by shaking (30 Hz) at room temperature (30°C) for 60 minutes to cause a reaction. Thereafter, the ball mill jar was uncapped to check the inside, and as a result, an oily substance with gray color was obtained.

Thereafter, 53.1 mg (0.5 mmol, 1.0 equiv) of benzaldehyde as an organic carbonyl compound was further added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. The dichloromethane was removed with an evaporator to obtain a reaction product (diphenylmethanol). The NMR yield was determined by 1H NMR to be 6%.

### [Examples 74 to 77 and Comparative Example 2]

The reaction was performed in the same manner as in Example 73 to obtain reaction products (diphenylmethanol), except that compounds in Table 5 were used as organic halogen compounds and ether compounds and the amounts of the components used were the same as in Example 73. The results are shown in Table 5 together with NMR yields.

**[Table 5]**

| | Organohalide | Ether compound | NMR yield (%) |
|---|---|---|---|
| Example 73 | Bromobenzene | Tetrahydrofuran | 94 |
| Example 74 | Chlorobenzene | Tetrahydrofuran | 74 |
| Example 75 | Iodebenzene | Tetrahydrofuran | 84 |
| Example 76 | Bromobenzene | Diethyl ether | 79 |
| Example 77 | Bromobenzene | Cyclopentyl methyl ether | 87 |
| Comparative Example 1 | Bromobenzene | - | 6 |
| Comparative Example 2 | Bromobenzene | Hexane | 1 |

### [Examples 78 to 83]

### <Method for producing alcohol, including reacting organohalide, metal or metal compound, and organic carbonyl compound (electrophile) with each other by mechanochemical process in presence of ether compound in amount of 0.5 to 10.0 equivalents relative to 1 equivalent of organohalide>

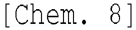

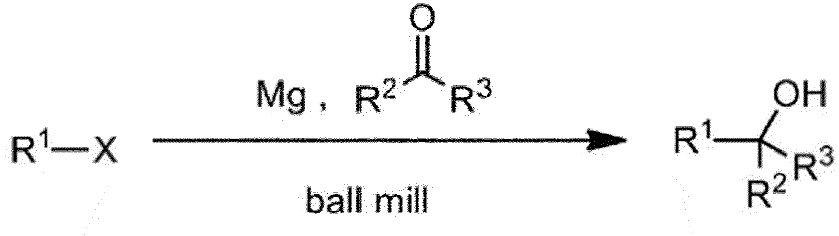

### [Example 78]

To a 5 mL stainless-steel ball mill jar containing stainless-steel balls 10 mm in diameter, 157.0 mg (1.00 mmol; 2.0 equiv) of bromobenzene as an organohalide, 60.8 mg (2.50 mmol; 5.0 equiv) of magnesium as a metal, 53.1 mg (0.50 mmol; 1.0 equiv) of benzaldehyde as an organic carbonyl compound, and 123 µL (1.5 mmol, 3.0 equiv) of tetrahydrofuran as an ether compound were added under air. The ball mill jar was capped, mounted to a ball mill, and stirred by shaking (30 Hz) at room temperature (30°C) for 15 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be 91%. The crude mixture was purified by silica gel column chromatography to isolate a target alcohol (diphenylmethanol). The isolated yield was 93%.

### [Examples 79 to 83]

The reaction was performed in the same manner as in Example 78 to obtain reaction products, except that compounds in Table 6 were used as organohalides and/or organic carbonyl compounds. The results are shown in Table 6 together with isolated yields and NMR yields.

**[Table 6]**

| Example | Organohalide | Electrophilic reagent | Reaction product | Isolated yield (%) | NMR yield (%) |
|---|---|---|---|---|---|
| 78 | 31a | 32a | 33aa | 93 | |
| 79 | 31a | 32b | 33ab | 92 | |
| 80 | 31a | 32c | 33ac | 86 | |
| 81 | 31b | 32a | 33ba | 72 | 91 |
| 82 | 31b | 32b | 33bb | 63 | 71 |
| 83 | 31b | 32c | 33bc | 52 | 73 |

The organohalides in Table 6 are as shown below.

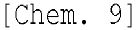

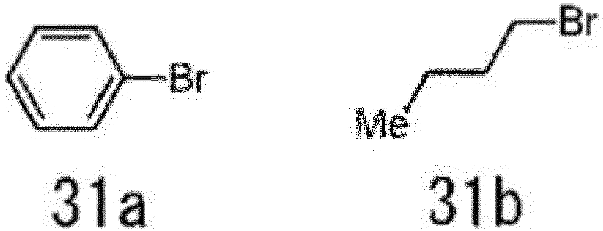

The organic carbonyl compounds in Table 6 are as shown below.

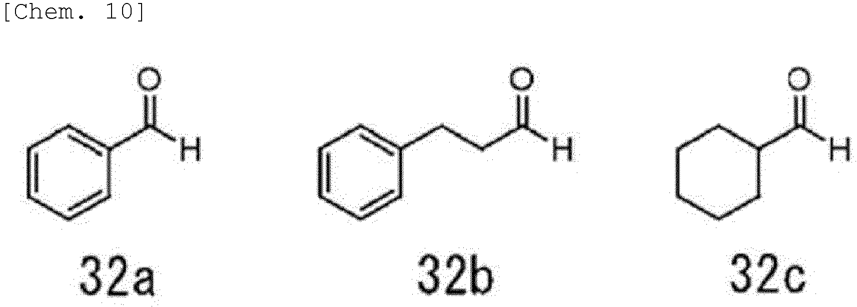

The reaction products in Table 6 are as shown below.

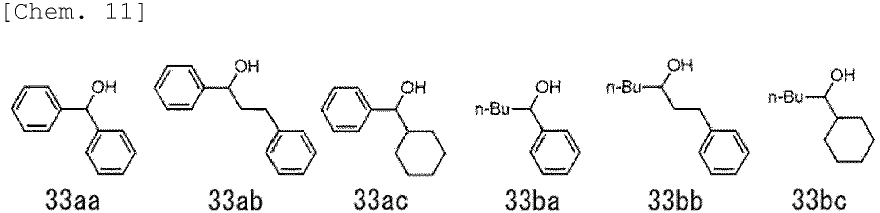

### [Examples 84 to 90]

<Addition reaction method including reacting organohalide and metal or metal compound with each other by mechanochemical process in presence of ether compound in amount of 0.5 to 10.0 equivalents relative to 1 equivalent of organohalide, and further adding electrophile and performing reaction by mechanochemical process>

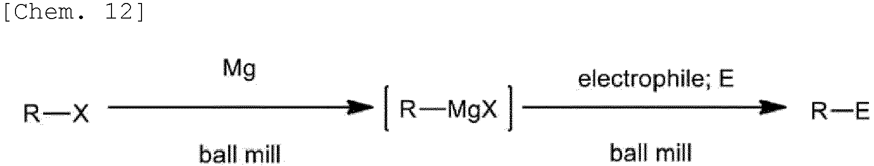

### [Example 84]

### <Reaction with Weinreb amide>

To a 5 mL stainless-steel ball mill jar containing stainless-steel balls 10 mm in diameter, 137.0 mg (1.00 mmol, 2.0 equiv) of 1-bromobutane (41a) as an organohalide, 60.8 mg (2.50 mmol, 5.0 equiv) of magnesium as a metal, and 123 µL (1.5 mmol, 3.0 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped, and 82.6 mg (0.5 mmol, 1.0 equiv) of N-methoxy-N-methylbenzamide (42a) as an electrophile (Weinreb amide) was added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be 69%. The crude mixture was purified by silica gel column chromatography to isolate target 1-phenylpentan-1-one (43aa). The isolated yield was 53%.

### [Example 85]

### <Reaction with Weinreb amide>

The reaction was performed in the same manner as in Example 84 to obtain target 1-phenylpentan-1-one (43aa), except that the amounts of the components used were as follows: organohalide, 1.5 equiv; metal, 1.5 equiv; ether compound, 3.0 equiv; and electrophile (Weinreb amide), 0.50 mmol (1.0 equiv). The NMR yield was 60%.

### [Example 86]

### <Reaction with ester>

To a 5 mL stainless-steel ball mill jar containing stainless-steel balls 10 mm in diameter, 137.0 mg (1.00 mmol, 3.0 equiv) of 1-bromobutane (41a) as an organohalide, 24.3 mg (1.00 mmol, 3.0 equiv) of magnesium as a metal, and 164 µL (2.0 mmol, 6.0 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped, and 44.9 mg (0.33 mmol, 1.0 equiv) of methyl benzoate (42b) was added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be 73%. The crude mixture was purified by silica gel column chromatography to isolate target 5-phenylnonan-5-ol (43ab). The isolated yield was 61%.

### [Example 87]

### <Reaction with nitrile>

To a 5 mL stainless-steel ball mill jar containing stainless-steel balls 10 mm in diameter, 137.0 mg (1.00 mmol, 2.0 equiv) of 1-bromobutane (41a) as an organohalide, 60.8 mg (2.50 mmol, 5.0 equiv) of magnesium as a metal, and 123 µL (1.5 mmol, 3.0 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped, and 76.6 mg (0.5 mmol, 1.0 equiv) of 2-naphthonitrile 4c was added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the crude mixture was purified by silica gel column chromatography to isolate target 1-(naphthalen-2-yl)pentan-1-one (43ac). The isolated yield was 52%.

### [Example 88]

The reaction was performed in the same manner as in Example 87 to obtain target 1-(naphthalen-2-yl)pentan-1-one (43ac), except that the amounts of the components used were as follows: organohalide, 1.5 equiv; metal, 1.5 equiv; ether compound, 3.0 equiv; and electrophile (nitrile), 0.50 mmol (1.0 equiv). The NMR yield was 40%.

### [Example 89]

### <Reaction with chlorosilane>

To a 5 mL stainless-steel ball mill jar containing stainless-steel balls 10 mm in diameter, 117.8 mg (0.75 mmol, 1.5 equiv) of bromobenzene (41b) as an organohalide, 18.2 mg (0.75 mmol, 1.5 equiv) of magnesium as a metal, and 123 µL (1.5 mmol, 3.0 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped, and 85.3 mg (0.5 mmol, 1.0 equiv) of chlorodimethylphenylsilane (42d) and 23.8 mg (0.125 mmol, 0.25 equiv) of copper iodide were added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be 86%. The crude mixture was purified by silica gel column chromatography to isolate target dimethyldiphenylsilane (43bd). The isolated yield was 62%.

### [Example 90]

### <Reaction with chlorosilane>

The reaction was performed in the same manner as in Example 89 to obtain target dimethyldiphenylsilane (43bd), except that the amounts of the components used were as follows: organohalide, 2.0 equiv; metal, 5.0 equiv; ether compound, 3.0 equiv; and electrophile (chlorosilane), 0.50 mmol (1.0 equiv). The isolated yield was 48%.

The organohalides (41a and 41b), the electrophiles (42a, 42b, 42c, and 42d), and the reaction products (43aa, 43ab, 43ac, and 43bd) used in Examples 84 to 90 are as shown below.

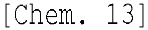

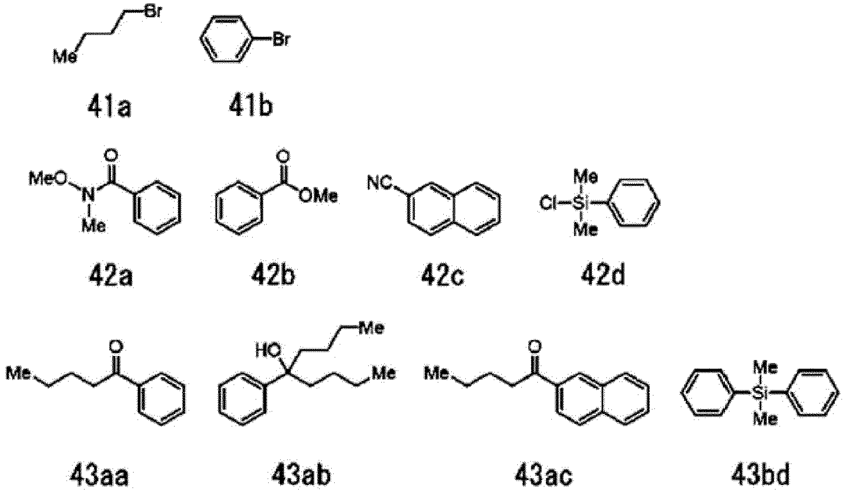

### [Examples 91 to 108]

### <Coupling method including reacting organohalide and metal or metal compound with each other by mechanochemical process in presence of ether compound in amount of 0.5 to 10.0 equivalents relative to 1 equivalent of organohalide, and further adding nickel catalyst and sulfonate ester compound and performing reaction by mechanochemical process>

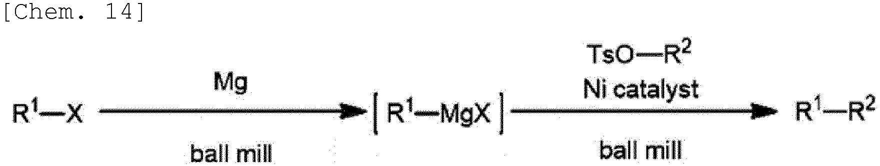

### [Example 91]

To a 5 mL stainless-steel ball mill jar containing stainless-steel balls 10 mm in diameter, 137 mg (1.0 mmol; 3.0 equiv) of 1-bromobutane (51a) as an organohalide, 60.8 mg (2.5 mmol; 7.5 equiv) of magnesium as a metal, and 123 µL (1.5 mmol; 4.5 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped, and 17.4 mg (0.033 mmol; 0.1 equiv) of 10 mol% [1,2-bis(diphenylphosphino)ethane]nickel(II) dichloride ((dppe)NiCl₂) as a nickel catalyst and 98.5 mg (0.33 mmol; 1.0 equiv) of 2-naphthyl tosylate (52a) as a sulfonate ester compound were added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the crude mixture was purified by silica gel column chromatography to obtain target 2-n-butyl-naphthalene. The isolated yield was 75%.

### [Examples 92 to 99]

The reaction was performed in the same manner as in Example 91 to obtain reaction products, except that compounds in Table 7 were used as organohalides and/or sulfonate ester compounds. The results are shown in Table 7 together with isolated yields.

### [Examples 100 to 108]

The reaction was performed in the same manner as in Example 91 to obtain reaction products, except that compounds in Table 7 were used as organohalides and/or sulfonate ester compounds and the amounts of the components used were as follows: organohalide, 1.5 equiv; metal (magnesium), 1.5 equiv; ether compound (tetrahydrofuran), 3.0 equiv; and sulfonate ester compound, 0.33 mmol (1.0 equiv). The results are shown in Table 7 together with isolated yields and NMR yields.

**[Table 7]**

| Example | Organohalide | Sulfonate ester compound | Reaction product | Isolated yield (%) | NMR yield (%) |
|---|---|---|---|---|---|
| 91 | 51a | 52a | 53aa | 75 | |
| 92 | 51a | 52b | 53ab | 70 | |
| 93 | 51b | 52a | 53ba | 88 | 99 |
| 94 | 51a | 52c | 53ac | 36 | |
| 95 | 51a | 52d | 53ad | 79 | |
| 96 | 51c | 52a | 53ca | 78 | >99 |
| 97 | 51d | 52a | 53da | 87 | |
| 98 | 51e | 52a | 53ea | 43 | 55 |
| 99 | 51f | 52a | 53fa | 40 | 53 |
| 100 | 51a | 52a | 53aa | | 35 |
| 101 | 51a | 52b | 53ab | | 44 |
| 102 | 51b | 52a | 53ba | | 31 |
| 103 | 51a | 52c | 53ac | | 26 |
| 104 | 51a | 52d | 53ad | | 41 |
| 105 | 51c | 52a | 53ca | | 16 |
| 106 | 51d | 52a | 53da | | 21 |
| 107 | 51e | 52a | 53ea | | 4 |
| 108 | 51f | 52a | 53fa | | <1 |

The organohalides in Table 7 are as shown below.

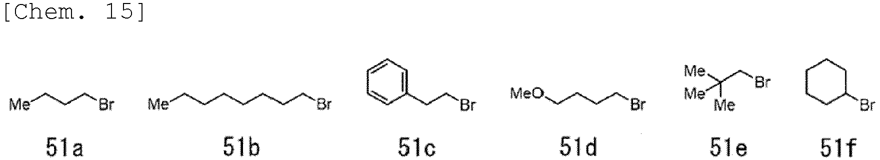

The sulfonate ester compounds in Table 7 are as shown below.

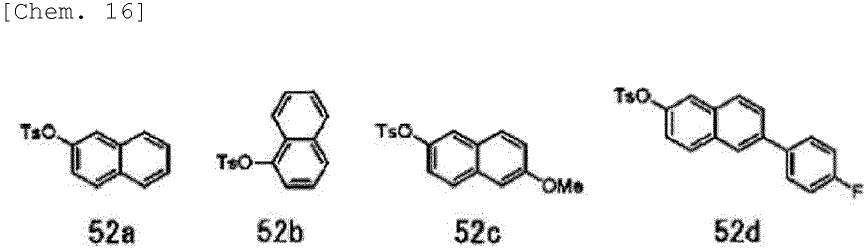

The reaction products in Table 7 are as shown below.

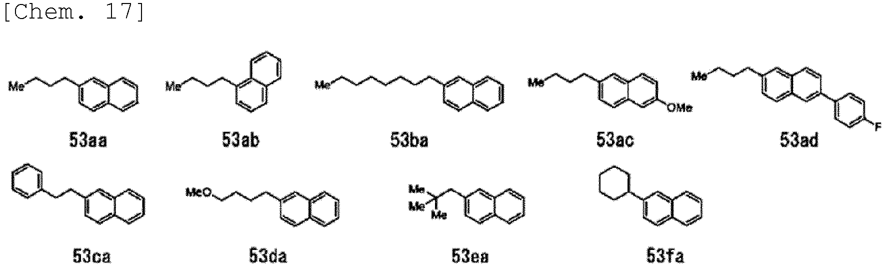

### [Example 109]

<Coupling method including reacting organohalide, metal or metal compound, sulfonate ester compound, and nickel catalyst by mechanochemical process in presence of ether compound in amount of 0.5 to 10.0 equivalents relative to 1 equivalent of organohalide>

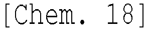

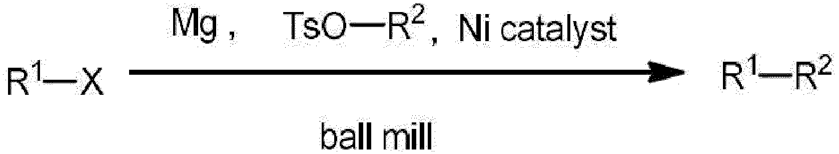

To a 5 mL stainless-steel ball mill jar containing stainless-steel balls 10 mm in diameter, 137.0 mg (1.0 mmol) of 1-bromobutane as an organohalide, 60.8 mg (2.5 mmol) of magnesium as a metal, 98.5 mg (0.33 mmol) of 2-naphthyl tosylate as a sulfonate ester compound (tosylal compound), 17.4 mg (0.033 mmol) of 10 mol% [1,2-bis(diphenylphosphino)ethane]nickel(II) dichloride ((dppe)NiCl₂) as a nickel catalyst, and 123 µL (1.5 mmol) of tetrahydrofuran as an ether compound were added under air. The ball mill jar was capped, mounted to a ball mill, and stirred by shaking (30 Hz) at room temperature (30°C) for 90 minutes to cause a reaction. After completion of the reaction, the reaction mixture was extracted with dichloromethane and dried over magnesium sulfate. Thereafter, inorganic salts were removed by filtration. After the dichloromethane was removed with an evaporator, the NMR yield of target 2-n-butyl-naphthalene was determined by 1H NMR. The NMR yield was 43%.

### [Examples 110 to 113]

<Addition reaction method including reacting organohalide and metal or metal compound with each other by mechanochemical process in presence of ether compound in amount of 0.5 to 10.0 equivalents relative to 1 equivalent of organohalide, and further adding conjugated enone compound and performing reaction by mechanochemical process>

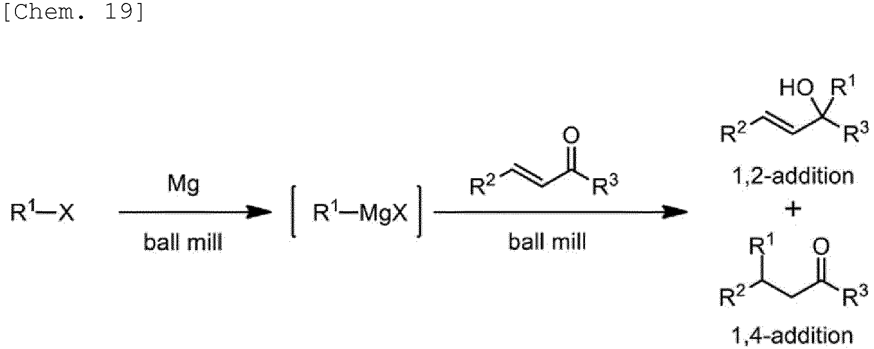

### [Example 110]

To a 5 mL stainless-steel ball mill jar containing stainless-steel balls 10 mm in diameter, 157.0 mg (1.00 mmol; 2.0 equiv) of bromobenzene as an organohalide, 60.8 mg (2.5 mmol; 5.0 equiv) of magnesium, and 123 µL (1.5 mmol; 3.0 equiv) of tetrahydrofuran as an ether compound were added under air. The ball mill jar was capped, mounted to a ball mill, and stirred by shaking (30 Hz) at room temperature (30°C) for 60 minutes to cause a reaction. Thereafter, the ball mill jar was uncapped, and 73.1 mg (0.50 mmol; 1.0 equiv) of benzylideneacetone as a conjugated enone compound was added. The ball mill jar was capped, mounted to the ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be as follows: 1,2-adduct, 59%; 1.4-adduct, 8%.

### [Example 111]

The reaction and purification were performed in the same manner as in Example 110 to obtain a reaction product, except that the amounts of the components used were as follows: organohalide (bromobenzene), 1.5 equiv; metal (magnesium), 1.5 equiv; ether compound (tetrahydrofuran), 3.0 equiv; and conjugated enone compound (benzylideneacetone), 0.50 mmol (1.0 equiv). The NMR yield was determined by 1H NMR to be as follows: 1,2-adduct, 56%; 1.4-adduct, 7%.

### [Example 112]

The reaction was performed in the same manner as in Example 110 except that 95.2 mg (0.5 mmol; 2.0 equiv) of copper iodide was added together with 36.5 mg (0.25 mmol; 1.0 equiv) of benzylideneacetone. The NMR yield was determined by 1H NMR to obtain a reaction mixture of 4% 1,2-addcuct and 46% 1.4-adduct.

### [Example 113]

The reaction was performed in the same manner as in Example 110 except that 184.9 mg (0.75 mmol; 1.5 equiv) of cerium chloride was added together with 73.1 mg (0.5 mmol; 1.0 equiv) of benzylideneacetone. The NMR yield was determined by 1H NMR to be as follows: 1,2-adduct, 37%; 1.4-adduct, 6%.

### [Comparative Example 3]

Bromobenzene in an amount of 117.8 mg (0.75 mmol; 1.5 equiv) and magnesium in an amount of 18.2 mg (0.75 mmol; 1.5 equiv) were mixed with 0.5 mL of tetrahydrofuran and allowed to react at room temperature for 60 minutes. Next, 73.1 mg (0.50 mmol; 1.0 equiv) of benzylideneacetone was added and allowed to react at room temperature for 60 minutes.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be as follows: 1,2-adduct, 75%; 1.4-adduct, 25%.

### [Example 114]

<Addition reaction method including reacting organohalide, metal or metal compound, and conjugated enone compound with each other by mechanochemical process in presence of ether compound in amount of 0.5 to 10.0 equivalents relative to 1 equivalent of organohalide>

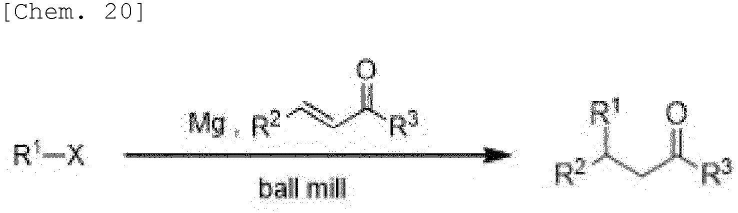

To a 5 mL stainless-steel ball mill jar containing stainless-steel balls 10 mm in diameter, 157.0 mg (1.00 mmol) of bromobenzene as an organohalide, 60.8 mg (2.5 mmol) of magnesium, 104.1 mg (0.5 mmol) of chalcone (1,3-diphenyl-2-propen-1-one) as a conjugated enone compound, and 123 µL (1.5 mmol) of tetrahydrofuran as an ether compound were added under air. The ball mill jar was capped, mounted to a ball mill, and stirred by shaking (30 Hz) at room temperature (30°C) for 60 minutes to cause a reaction.

After completion of the reaction, the reaction mixture was extracted with dichloromethane and dried over magnesium sulfate. Thereafter, inorganic salts were removed by filtration. After the dichloromethane was removed with an evaporator, the NMR yield was determined by 1H NMR to be 60%.

### [Example 115 and Comparative Example 4]

### <Superiority of mechanochemical process for halide with low solubility>

### [Example 115]

To a 5 mL stainless-steel ball mill jar containing stainless-steel balls 10 mm in diameter, 309.2 mg (1.00 mmol; 2.0 equiv) of 4-bromo-p-terphenyl (71a) as an organohalide, 60.8 mg (2.50 mmol; 5.0 equiv) of magnesium as a metal, and 123 µL (1.5 mmol, 3.0 equiv) of tetrahydrofuran as an ether compound were added under air. The ball mill jar was capped, mounted to a ball mill, and stirred by shaking (30 Hz) for 60 minutes while being heated with a heat gun so as to have an internal temperature of 70°C to cause a reaction. Thereafter, the ball mill jar was uncapped, and 67.1 mg (0.50 mmol; 1.0 equiv) of 3-phenylpropionaldehyde (72a) as an organic carbonyl compound was added. The ball mill jar was capped, mounted to the ball mill, and stirred by shaking (30 Hz) at room temperature (30°C) for 60 minutes in air to cause a reaction.

After completion of the reaction, the reaction mixture (73aa) was extracted with dichloromethane and dried over magnesium sulfate. Thereafter, inorganic salts were removed by filtration. After the dichloromethane was removed with an evaporator, the NMR yield was determined by 1H NMR to be 42%.

### [Comparative Example 4]

To a reaction vessel, 309.2 mg (1.00 mmol; 2.0 equiv) of 4-bromo-p-terphenyl (71a) as an organohalide, 60.8 mg (2.50 mmol; 5.0 equiv) of magnesium as a metal, and 3 mL (0.33 mol) of tetrahydrofuran as an ether compound were added, and allowed to react in a nitrogen atmosphere for 24 hours under reflux. Thereafter, 67.1 mg (0.50 mmol; 1.0 equiv) of 3-phenylpropionaldehyde (72a) as an organic carbonyl compound was added to the reaction vessel and allowed to react in a nitrogen atmosphere at room temperature (30°C) for 60 minutes.

After completion of the reaction, the reaction mixture (73aa) was extracted with dichloromethane and dried over magnesium sulfate. Thereafter, inorganic salts were removed by filtration. After the dichloromethane was removed with an evaporator, the NMR yield was determined by 1H NMR to be less than 1%.

The organohalide (4-bromo-p-terphenyl: 71a), the organic carbonyl compound (3-phenylpropionaldehyde: 72a), and the reaction product (73aa) used in Example 115 and Comparative Example 4 are as shown below.

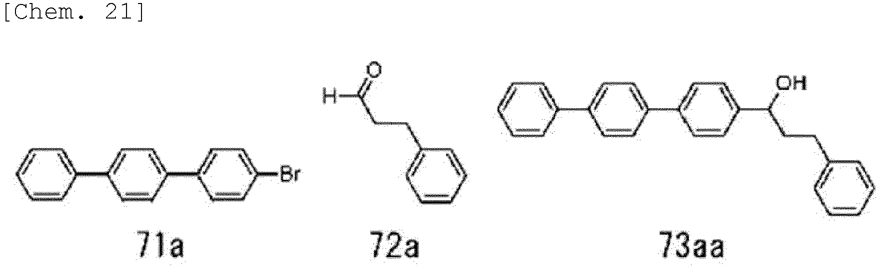

### [Example 116 and Comparative Example 5]

### <Identification of organometallic nucleophile using NEXAFS spectrum>

### [Example 116]

To a 5 mL stainless-steel ball mill jar containing stainless-steel balls 10 mm in diameter, 157.0 mg (1.00 mmol; 1.0 equiv) of bromobenzene as an organohalide, 24.3 mg (1.00 mmol; 1.0 equiv) of magnesium as a metal, and 164 µL (2.00 mmol, 2.0 equiv) of tetrahydrofuran as an ether compound were added under air. The ball mill jar was capped, mounted to a ball mill, and stirred by shaking (30 Hz) at room temperature (30°C) for 60 minutes to cause a reaction.

After completion of the reaction, the reaction mixture was purified to obtain a target organometallic nucleophile PhMgBr (phenylmagnesium bromide).

PhMgBr prepared mechanochemically in Example 116 and PhMgBr prepared in solution (16% tetrahydrofuran solution, about 1 mol/L; commercially available product) were measured for their Mg K-edge NEXAFS (near-edge x-ray absorption fine structure (NEXAFS) at the Mg-K absorption edge) and C K-edge NEXAFS (near-edge x-ray absorption fine structure at the C-K absorption edge), and the spectra were compared with each other.

The measurement of Mg K-edge NEXAFS was performed using the soft X-ray beamline BL2A of UVSOR-III synchrotron. A gummy sample of PhMgBr prepared mechanochemically was attached to high-purity indium foil and fixed to a cup-shaped sample holder. For a sample of PhMgBr prepared in solution, a 1.0 M THF solution of PhMgBr without MgBr₂ precipitation was dropped on high-purity indium foil and dried.

The sample holder was fixed to a manipulator and placed in a vacuum chamber evacuated to 1 × 10⁻⁶ Pa or lower. The sample preparation and the placement operation were carefully performed in an argon atmosphere.

The Mg K-edge NEXAFS spectra (1250-1400 eV) were acquired in total electron yields (TEYs) by measuring the drain current of the samples in an argon atmosphere. The energy resolution of the soft X-rays incident on the Mg K-edge was set to 0.2 eV in the range from 1300 to 1330 eV and to 1.0 eV in the ranges from 1250 to 1300 eV and from 1330 to 1400 eV.

The C K-edge NEXAFS measurement was performed using the soft X-ray beamline BL3U of UVSOR-III synchrotron. The PhMgBr samples were all fixed to a stainless-steel sample holder and placed in a vacuum chamber evacuated to 1 × 10⁻⁶ Pa or lower as in the Mg K-edge NEXAFS measurement.

The C K-edge NEXASF spectra (280-300 eV) were acquired in total electron yields (TEYs) by measuring the drain current of the samples in an argon atmosphere. The energy resolution of the soft X-rays incident on the C K-edge was set to 0.05 eV in the range from 283 to 293 eV and to 0.2 eV in the ranges from 280 to 283 eV and from 293 to 300 eV.

Data processing such as subtraction of indium foil background, baseline correction, and normalization of the resulting spectra was performed using the Athena program.

The formation of divalent cationic Mg²⁺ species can be clearly confirmed from a high-energy shift from the Mg⁰ absorption edge (1302.6 eV) of a starting magnesium flake to the Mg-K absorption edge (1307.4 eV) (Fig. 1).

The Mg-K NEXAFS spectra and the C-K NEXAFS spectra of PhMgBr prepared mechanochemically and PhMgBr prepared in solution are well-approximated as shown in Fig. 1 and Fig. 2, which confirms that substantially the same organomagnesium species (organometallic nucleophile) having a carbon-magnesium bond is formed also by the mechanochemical process as well as by the conventional production process (production in THF solution).

In addition, intense 1s-π* transition peaks are observed at 285.7 eV and 287.7 eV in the C-K NEXAFS spectra shown in Fig. 2, which supports the formation of a carbon-magnesium bond resulting from transformation of a C-Br bond of bromobenzene as a starting material.

Fig. 3 shows that when THF (ether compound) is contained in a complex, the cubic structure of a halogenated organometallic compound not containing THF (r = 0) transforms into an open structure with higher reactivity, and coordination of THF to Mg atoms accompanied by Mg-Br bond scission occurs.

It can be seen from Examples 1 to 116 and Comparative Examples 1 to 5 that the method for producing an organometallic nucleophile, the method for producing an alcohol, the addition reaction method, and the coupling reaction method according to the present invention are industrially very useful inventions that can achieve reductions in environmental load and, furthermore, reductions in cost by simplified methods under simplified reaction conditions.

For example, it can be seen from Examples 73 to 77 and Comparative Examples 1 and 2 that the use of an ether compound is significantly advantageous.

For example, it can be seen from Example 115 and Comparative Example 4 that an organometallic nucleophile can be obtained by a simplified method under simplified reaction conditions from a compound which has previously been difficult to form an organometallic nucleophile, which is significantly advantageous.

### [Example 117]

### <Mechanochemical synthesis of calcium carbon nucleophile ("Heavy" Grignard reagent)>

To a 1.5 mL stainless-steel ball mill jar containing two stainless-steel balls 5 mm in diameter, 127 mg (0.5 mmol; 1 equiv) of 1-iodonaphthalene as an organohalide, 30 mg (0.75 mmol; 1.5 equiv) of calcium powder (purity, 99%; particle size, 5 mm or less) as a metal, and 196 µL (2 mmol, 4.0 equiv) of tetrahydropyran (THP) as an ether compound were added under air. The ball mill jar was capped, mounted to a ball mill, and stirred by shaking (30 Hz) for 60 minutes while being heated with a heat gun so as to have an internal temperature of 60°C to cause a reaction. Thereafter, the ball mill jar was uncapped, and 1 mL (1 mmol; 2 equiv) of 1.0 M hydrochloric acid was added. Naphthalene, the reaction product, was extracted with dichloromethane and dried over magnesium sulfate. After filtration, the dichloromethane was removed with an evaporator, and the NMR yield was determined by 1H NMR to be 32%. The conversion rate was more than 99%.

### [Examples 118 to 135]

### <Mechanochemical synthesis of calcium carbon nucleophile ("Heavy" Grignard reagent) and its application to alkylation reaction>

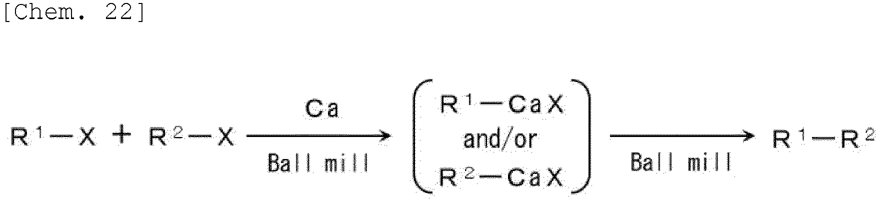

### [Example 118]

To a 1.5 mL stainless-steel ball mill jar containing two stainless-steel balls 5 mm in diameter, 127 mg (0.5 mmol; 1 equiv) of 1-iodonaphthalene as an aromatic halide and 312 mg (2 mmol; 4 equiv) of iodoethane as an aliphatic halide, 30 mg (0.75 mmol; 1.5 equiv) of calcium powder (purity, 99%; particle size, 5 mm or less) as a metal, and 196 µL (2 mmol, 4.0 equiv) of tetrahydropyran (THP) as an ether compound were added under air. The ball mill jar was capped, mounted to a ball mill, and stirred by shaking (30 Hz) for 60 minutes while being heated with a heat gun so as to have an internal temperature of 60°C to cause a reaction.

After completion of the reaction, 1-ethylnaphthalene, the reaction product, was extracted with dichloromethane and dried over magnesium sulfate. After filtration, the dichloromethane was removed with an evaporator to obtain a reactive product. The NMR yield was determined by 1H NMR to be 80%, and the isolated yield was determined to be 69%.

### [Example 119]

The reaction and purification were performed in the same manner as in Example 118 to obtain a reaction product, except that 18 mg (0.75 mmol; 1.5 equiv) of magnesium powder as a metal was used. The NMR yield was determined by 1H NMR to be 15%.

This shows that even when the yield is low and the reaction is difficult with magnesium, the reaction can readily proceed in high yield when calcium is used.

### [Examples 120 to 135]

The reaction was performed in the same manner as in Example 118 to obtain reaction products, except that compounds in Table 8 were used as aromatic halides and aliphatic halides. The results are shown in Table 8 together with isolated yields and NMR yields.

**[Table 8]**

| Example | Aromatic halide | Aliphatic halide | Reaction product | Isolated yield (%) | NMR yield (%) |
|---|---|---|---|---|---|
| 118 | 101a | 102a | 103a | 80 | 69 |
| 119 (Mg) | 101a | 102a | 103a | | 15 |
| 120 | 101b | 102a | 103b | 41 | 65 |
| 121 | 101c | 102a | 103c | 52 | 63 |
| 122 | 101d | 102a | 103d | 67 | 74 |
| 123 | 101e | 102a | 103e | 73 | 80 |
| 124 | 101f | 102a | 103f | 58 | 73 |
| 125 | 101g | 102a | 103g | 40 | 58 |
| 126 | 101h | 102a | 103h | 64 | 72 |
| 127 | 101i | 102a | 103i | 80 | 82 |
| 128 | 101a | 102b | 103j | 52 | 77 |
| 129 | 101a | 102c | 103k | 65 | 75 |
| 130 | 101a | 102d | 103l | 58 | 69 |
| 131 | 101a | 102e | 103m | 62 | 73 |
| 132 | 101a | 102f | 103n | | 63 |
| 133 | 101a | 102g | 103o | | 57 |
| 134 | 101a | 102h | 103p | 47 | 54 |
| 135 | 101a | 102i | 103q | | 63 |

The aromatic halides in Table 8 are as shown below.

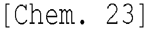

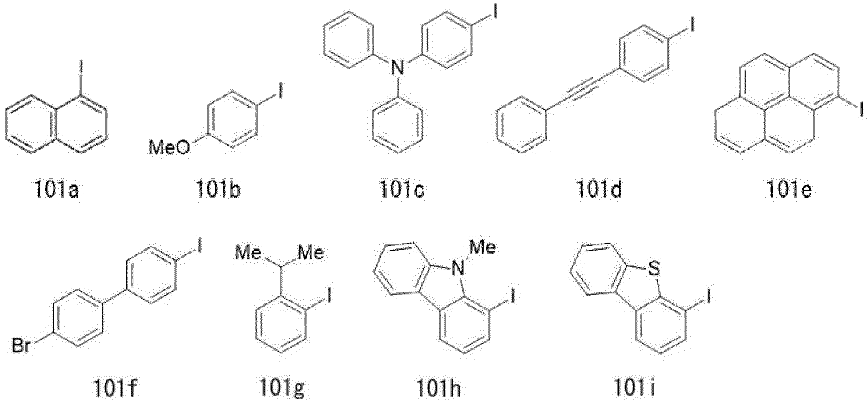

The aliphatic halides in Table 8 are as shown below.

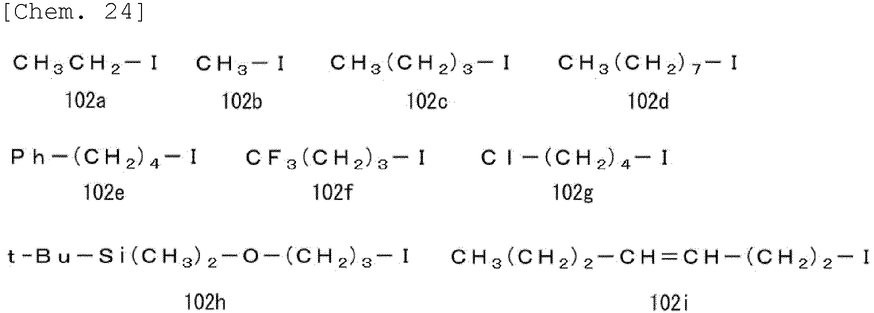

The reaction products in Table 8 are as shown below.

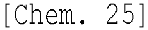

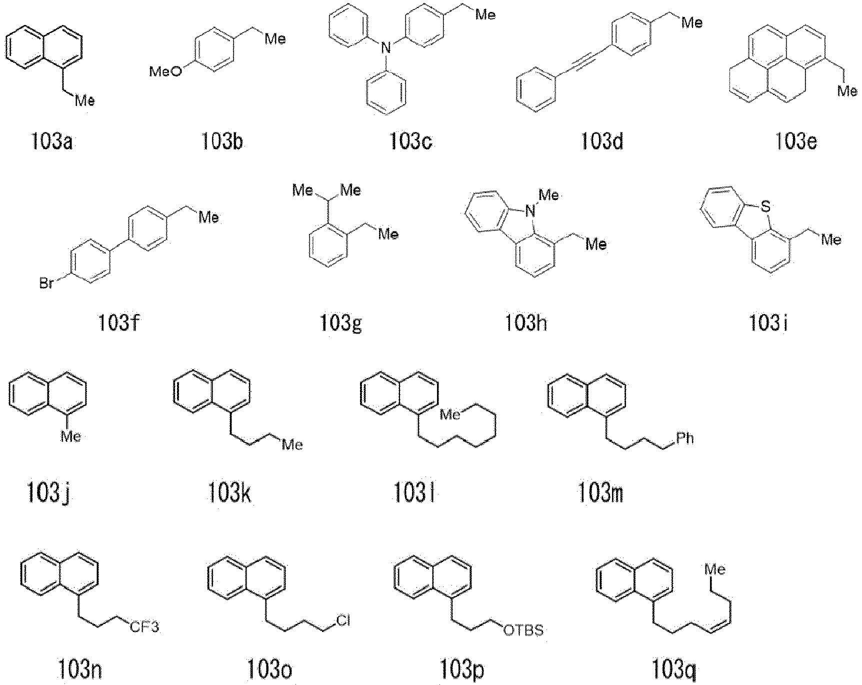

### [Examples 136 to 141]

### <Mechanochemical synthesis of organomanganese reagent>

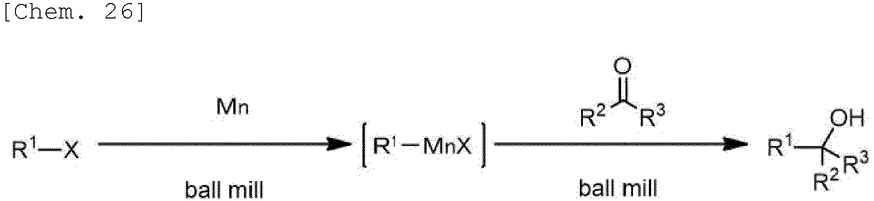

### [Example 136]

To a 5 mL stainless-steel ball mill jar containing stainless-steel balls 10 mm in diameter, 1 mmol of 1-fluoro-2-iodobenzene as an aromatic halide, 3 mmol of manganese powder as a metal, and 2 mmol of tetrahydrofuran (THF) as an ether compound were added under air. The ball mill jar was capped, mounted to a ball mill, and stirred by shaking (30 Hz) for 180 minutes while being heated with a heat gun so as to have an internal temperature of 35°C to cause a reaction. Thereafter, the ball mill jar was uncapped. In the ball mill jar, a paste-like substance was formed, and no manganese powder was observed. Subsequently, 2 mmol of 1-octanal (CH₃(CH₂)₆CHO) was added into the ball mill jar. The ball mill jar was capped, mounted to the ball mill, and stirred by shaking (30 Hz) for 30 minutes at an internal temperature of 35°C in air to cause a reaction.

After completion of the reaction, the reaction product was extracted with dichloromethane and dried over magnesium sulfate. After filtration, the dichloromethane was removed with an evaporator to obtain a reactive product. The NMR yield was determined by 1H NMR to be 50%.

### [Examples 137 to 141]

The reaction was performed in the same manner as in Example 136 to obtain reaction products, except that compounds in Table 9 were used as aromatic halides and the internal temperature was 70°C. The results are shown in Table 9 together with NMR yields.

**[Table 9]**

| Example | Aromatic halide | Reaction product | NMR yield (%) |
|---|---|---|---|
| 136 | 201a | 202a | 50 |
| 137 | 201b | 202b | 69 |
| 138 | 201c | 202c | 58 |
| 139 | 201d | 202c | 39 |
| 140 | 201e | 202d | 59 |
| 141 | 201f | 202e | 36 |

The aromatic halides in Table 9 are as shown below.

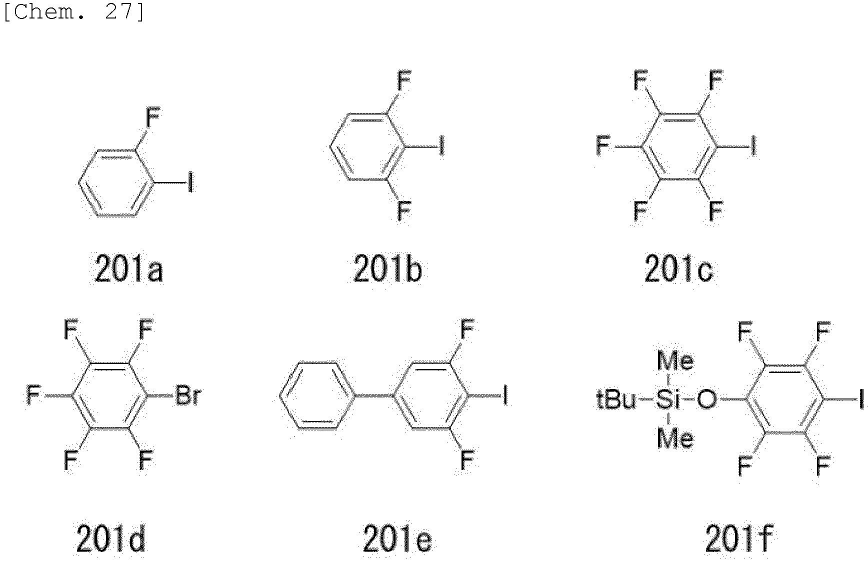

The reaction products in Table 9 are as shown below.

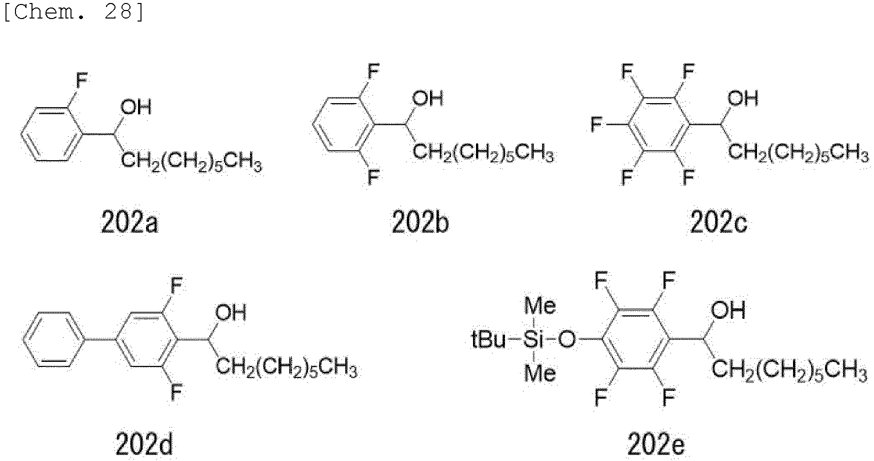

### [Comparative Examples 6 to 9]

### <Preparation of organomanganese reagent under solution conditions>

### [Comparative Example 6]

To a flask, 1 mmol of 1-fluoro-2-iodobenzene as an aromatic halide, 3 mmol (3 equiv) of manganese powder as a metal, and 0.5 mol of tetrahydrofuran (THF) as an ether compound were added. After stirring at a system temperature of 35°C for 1.5 hours in a nitrogen atmosphere, 2 mmol (2 equiv) of 1-octanal was added, and stirring was performed at 25°C for 30 minutes. The NMR yield of a reaction product (202a in Table 9) was determined by 1H NMR, but the reaction product was not detected. The recovery rate of the aromatic halide was 79%, confirming that the reaction between the manganese powder and the aromatic halide did not occur.

### [Comparative Examples 7 to 9]

The NMR yield of a reaction product (202a in Table 9) was determined by 1H NMR in the same manner as in Comparative Example 6 except that 1-octanal was added after stirring at a system temperature of 70°C for 1.5 hours (Comparative Example 7), stirring at a system temperature of 35°C for 24 hours (Comparative Example 8), or stirring at a system temperature of 70°C for 24 hours (Comparative Example 9) in a nitrogen atmosphere, but the reaction product was not detected in any cases. The recovery rates of the aromatic halide were 91% (Comparative Example 7), 77% (Comparative Example 8), and 85% (Comparative Example 9), confirming that the reaction between the manganese powder and the aromatic halide did not occur.

### [Examples 142 to 150]

### <Effect of ether compound in mechanochemical synthesis of organomanganese reagent>

### [Example 142]

To a 5 mL stainless-steel ball mill jar containing stainless-steel balls 10 mm in diameter, 1 mmol of pentafluoroiodobenzene as an aromatic halide, 3 mmol of manganese powder as a metal, and 207 µL of dimethyl ether (2 mmol (2 equiv); compound 221a below) as an ether compound were added under air. The ball mill jar was capped, mounted to a ball mill, and stirred by shaking (30 Hz) for 90 minutes while being heated with a heat gun so as to have an internal temperature of 35°C to cause a reaction. Thereafter, the ball mill jar was uncapped. In the ball mill jar, a paste-like substance was formed, and no manganese powder was observed. Subsequently, 2 mmol (2 equiv) of 1-octanal was added into the ball mill jar. The ball mill jar was capped, mounted to the ball mill, and stirred by shaking (30 Hz) for 30 minutes at an internal temperature of 35°C in air to cause a reaction.

After completion of the reaction, the reaction product was extracted with dichloromethane and dried over magnesium sulfate. After filtration, the dichloromethane was removed with an evaporator to obtain a reactive product. The NMR yield was determined by 1H NMR to be 40%.

### [Examples 143 to 150]

The reaction was performed in the same manner as in Example 142 to obtain reaction products, except that compounds in Table 10 were used in an amount of 2 equiv (2 mmol) as ether compounds. The results are shown in Table 10 together with NMR yields.

**[Table 10]**

| Example | Ether compound | NMR yield (%) |
|---|---|---|
| 142 | 203a | 40 |
| 143 | 203b | 54 |
| 144 | 203c | 26 |
| 145 | 203d | 64 |
| 146 | 203e | 64 |
| 147 | 203f | 57 |
| 148 | 203g | 77 |
| 149 | 203h | 66 |
| 150 | 203i | 10 |

The ether compounds in Table 10 are as shown below.

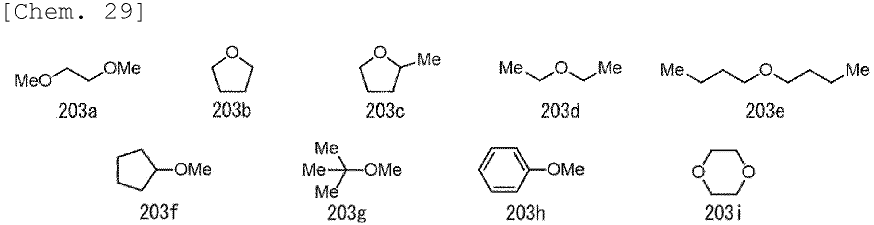

Examples of known methods for obtaining an organomanganese reagent by a reaction between metallic manganese and a halide include the following methods (i) and (ii) .

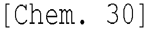

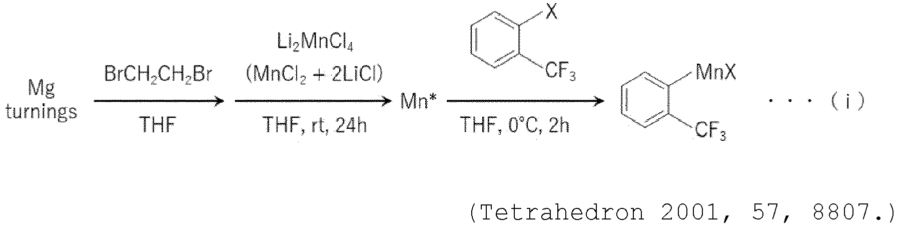

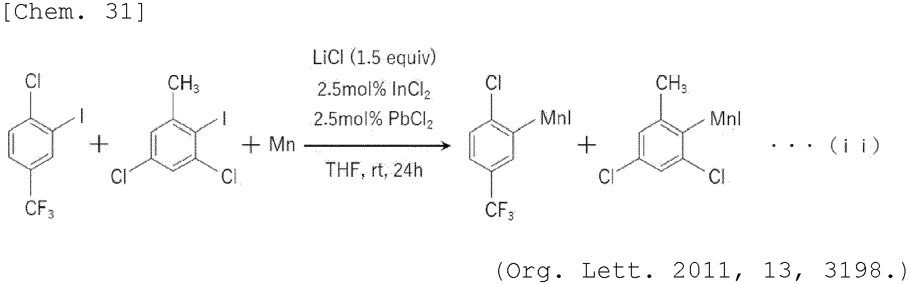

(i) is a method involving reduction of a manganese halide MnX₂, and (ii) is a preparation method using metallic Mn and additives, each method being a preparation method using a highly reactive activator or additives difficult to handle. By contrast, the method for preparing an organomanganese reagent by a mechanochemical process according to the present invention is a simplified method not using an activator or additives difficult to handle, and is extremely advantageous.

In addition, as shown in Comparative Examples 6 to 9, when metallic Mn and a halide are mixed with each other in a conventional solution system, no reaction occurs, and a target compound cannot be obtained. Also in this respect, the method for preparing an organomanganese reagent by a mechanochemical process according to the present invention is extremely advantageous.

## Claims

1. A method for producing an organometallic nucleophile, comprising reacting an organohalide and a metal or metal compound with each other by a mechanochemical process in the presence of an ether compound in an amount of 0.5 to 10.0 equivalents relative to 1 equivalent of the organohalide.

2. A method for producing an alcohol, comprising reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding an organic carbonyl compound and performing a reaction by the mechanochemical process.

3. A method for producing an alcohol, comprising reacting an organohalide, a metal or metal compound, and an organic carbonyl compound with each other by a mechanochemical process in the presence of an ether compound in an amount of 0.5 to 10.0 equivalents relative to 1 equivalent of the organohalide.

4. An addition reaction method comprising reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding an electrophile and performing a reaction by the mechanochemical process.

5. An addition reaction method comprising reacting an organohalide, a metal or metal compound, and an electrophile with each other by a mechanochemical process in the presence of an ether compound in an amount of 0.5 to 10.0 equivalents relative to 1 equivalent of the organohalide.

6. A coupling reaction method comprising reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding a nickel catalyst and a sulfonate ester compound and performing a reaction by the mechanochemical process.

7. A coupling reaction method comprising reacting an organohalide, a metal or metal compound, and a sulfonate ester compound with each other by a mechanochemical process in the presence of a nickel catalyst and an ether compound in an amount of 0.5 to 10.0 equivalents relative to 1 equivalent of the organohalide.

8. An addition reaction method comprising reacting an organohalide and a metal or metal compound with each other by a mechanochemical process in the presence of an ether compound in an amount of 0.5 to 10.0 equivalents relative to 1 equivalent of the organohalide to produce an organometallic nucleophile, and further adding a conjugated enone compound and performing a reaction by the mechanochemical process.

9. An addition reaction method comprising reacting an organohalide, a metal or metal compound, and a conjugated enone compound with each other by a mechanochemical process in the presence of an ether compound in an amount of 0.5 to 10.0 equivalents relative to 1 equivalent of the organohalide.

10. A composition obtained by the method according to Claim 1, comprising a halogenated organometallic compound and an ether compound, wherein a content of the ether compound is 0.5 to 10 equivalents relative to 1 equivalent of the halogenated organometallic compound.

11. A halogenated organometallic compound-ether compound complex obtained by the method according to Claim 1, comprising at least a halogenated organometallic compound and an ether compound, wherein the complex is represented by formula (A):
[R¹¹-(MX)ₚ]_{q}-rE (A)
(where R¹¹ is a p-valent organic group derived from the halogenated organometallic compound, M is a metal derived from the halogenated organometallic compound, X is a halogen, and E is the ether compound; a plurality of R¹¹'s, M's, X's, or E's, if present, may be the same or different; and p is an integer of 1 or greater, q is a number of 2 or greater, and r is a number greater than 0).

12. A halogenated organometallic compound-ether compound complex comprising at least a halogenated organometallic compound and an ether compound, wherein the complex is represented by formula (A):
[R¹¹-(MX)ₚ]_{q}-rE (A)
(where R¹¹ is a p-valent organic group derived from the halogenated organometallic compound, M is a metal derived from the halogenated organometallic compound, X is a halogen, and E is the ether compound; a plurality of R¹¹'s, M's, X's, or E's, if present, may be the same or different; and p is an integer of 1 or greater, q is a number of 2 or greater, and r is a number greater than 0).

13. An organometallic nucleophile obtained by the method according to Claim 1 using an organohalide having a solubility at 20°C in an ether compound of less than 1.0 mol/L.
